# EUROPEAN PATENT APPLICATION

(11) **EP 2 540 711 A1**
(43) Date of publication of application: **02.01.2013**
(21) Application number: 10846670.7
(22) Date of filing: 25.02.2010
(51) Int. Cl.: C07D 293/06, A61K 31/095, A61P 3/04, A61P 3/06, A61P 9/10

(54) **SELENALZOLE DERIVATIVE HAVING LIGAND WHICH ACTIVATES PEROXISOME PROLIFERATOR ACTIVATED RECEPTOR (PPAR), PREPARING METHOD THEREOF AND USAGE OF THE CHEMICAL COMPOUNDS**

(71) Applicant: SNU R & DB Foundation, Gwanak-gu, Seoul 151-015 (KR)
(72) Inventor: KANG, Heonjoong, Seongnam-si Gyeonggi-do 463-749 (KR); CHIN, Jungwook, Seoul 138-791 (KR); LEE, Jaehwan, Seoul 151-019 (KR)
(74) Representative: Zinnecker, Armin
(86) International application number: PCT/KR2010/001204
(87) International publication number: WO 2011/105643

(57) **Abstract**

Provided are a novel selenazole derivative which activates peroxisome proliferator-activated receptor (PPAR), a hydrate thereof, a solvate thereof, a stereoisomer thereof and a pharmaceutically acceptable salt thereof, a method for preparing the same, and a pharmaceutical composition, a cosmetic composition, a functional food composition, a functional drink composition and an animal feed composition containing the same.

## Description

### [Technical Field]

The present invention relates to a selenazole derivative compound represented by Chemical Formula I, which is useful as a ligand activating peroxisome proliferator-activated receptor (PPAR) that may be used for treatment of obesity, hyperlipemia, fatty liver, atherosclerosis and diabetes, a hydrate thereof, a solvate thereof, a stereoisomer thereof and a pharmaceutically acceptable salt thereof, and a pharmaceutical composition, a cosmetic composition, a functional food composition, a functional drink composition and an animal feed composition containing the same:

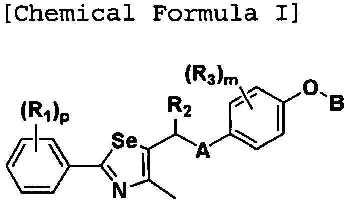

### [Background Art]

Peroxisome proliferator-activated receptors (PPARs) are nuclear receptors. Three subtypes PPARα, PPARγ and PPARδ have been identified *(*Nature, 1990, 347, p. 645-650, Proc. Natl. Acad. Sci. USA 1994, 91, p. 7335-7359). PPARα, PPARγ and PPARδ have different functions and are expressed indifferent tissues. PPARα is expressedmainly inheart, kidney, skeletal muscle and colon tissues in human *(*Mol. Pharmacol. 1998, 53, p. 14-22, Toxicol. Lett. 1999, 110, p. 119-127, J. Biol. Chem. 1998, 273, p. 16710-16714), and is involved in β-oxidation in peroxisome and mitochondria *(*Biol. Cell. 1993, 77, p. 67-76, J. Biol. Chem. 1997, 272, p. 27307-27312). PPARγ is weakly expressed in skeletal muscle tissue but is highly expressed in adipose tissue. It is known to be involved in differentiation of fat cells, storing of energy as fat, and regulation of insulin and sugar homeostasis *(*Moll. Cell. 1999, 4, p. 585-594, p. 597-609, p. 611-617). PPARδ is evolutionally conserved in mammals, including human, rodents and ascidians. It was identified as PPARβ in *Xenopus laevis (*Cell 1992, 68, p. 879-887) and, inhuman, as NUCI *(*Mol. Endocrinol. 1992, 6, p. 1634-1641), PPARδ *(*Proc. Natl. Acad. Sci. USA 1994, 91, p. 7355-7359), NUCI *(*Biochem. Biophys. Res. Commun. 1993, 196, p. 671-677) or FAAR *(*J. Bio. Chem. 1995, 270, p. 2367-2371). Recently, its name was unified as PPARδ. Inhuman, PPARδ is known to exist in chromosome 6p21.1-p21.2. Inmouse, mRNA of PPARδ is found in various areas, but the quantity is lower than that of PPARα or PPARγ *(*Endocrinology 1996, 137, p. 354-366, J. Bio. Chem. 1995, 270, p. 2367-2371, Endocrinology 1996, 137, p. 354-366). According to researches until now, PPARδ plays a very important role in the expression of gametes (Genes Dev. 1999, 13, p. 1561-1574). Also, it is known to be involved in differentiation of nerve cells in the central nervous system (CNS) *(*J. Chem. Neuroanat. 2000, 19, p. 225-232), wound healing through antiphlogistic action *(*Genes Dev. 2001, 15, p. 3263-3277, Proc. Natl. Acad. Sci. USA 2003, 100, p. 6295-6296), or the like. A recent study revealed that PPARδ is involved in differentiation of fat cells and metabolism of fat *(*Proc. Natl. Acad. Sci. USA 2002, 99, p. 303-308, Mol. Cell. Biol. 2000, 20, p. 5119-5128). It was found out that PPARδ activates expression of critical genes involved in β-oxidation and uncoupling proteins (UCPs), which are involved in energy metabolism, during breakdown of fatty acid, and thereby improves obesity and endurance *(*Nature 2000, 406, p. 415-418, Cell 2003, 113, p. 159-170, PLoS Biology 2004, 2, e294, Cell, 2008, 134, 405415). Also, activation of PPARδ results in increased HDL level and improved type 2 diabetes without change inbodyweight *(*Proc. Natl. Acad. Sci. USA2001, 98, p. 5306-5311, 2003, 100, p. 15924-15929, 2006, 103, p. 3444-3449), and enables treatment of atherosclerosis by inhibiting atherosclerosis -related genes (Science, 2003, 302, p. 453-457, PNAS, 2008, 105, 42714276). Accordingly, regulation of fat metabolism using PPARδ provides an important tool for treating obesity, diabetes, hyperlipemia and atherosclerosis.

### [Disclosure]

### [Technical Problem]

An object of the present invention is to provide a novel compound which selectively activates PPAR δ. Another object of the present invention is to provide a pharmaceutical composition, a cosmetic composition, a functional food composition, a functional drink composition and an animal feed composition containing the novel compound according to the present invention.

### [Technical Solution]

The present invention provides a selenazole derivative compound represented by Chemical Formula I, which activates peroxisome proliferator-activated receptor (PPAR), a solvate thereof, a stereoisomer thereof and a pharmaceutically acceptable salt thereof, a method for preparing the same, and a pharmaceutical composition, a cosmetic composition, a functional food composition, a functional drink composition and an animal feed composition containing the same: 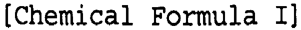 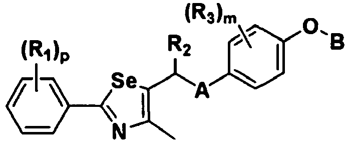 wherein A represents O, NR, S, S(=O), S(=O)₂ or Se; B represents hydrogen or 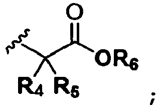 R₁ represents hydrogen, C1-C8 alkyl or halogen;
R₂ represents hydrogen, C1-C8 alkyl, 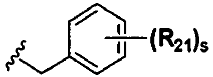 or 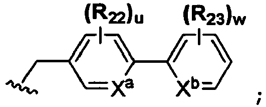 X^{a} and X^{b} independently represent CR or N; R represents hydrogen or C1-C8 alkyl; R₃ represents hydrogen, C1-C8 alkyl or halogen; R₄ and R₅ independently represent hydrogen, halogen or C1-C8 alkyl; R₆ represents hydrogen, halogen, C1-C8 alkyl, C2-C7 alkenyl, allyl, an alkali metal, an alkaline earth metal or a pharmaceutically acceptable organic salt; R₂₁, R₂₂, and R₂₃ independently represent hydrogen, halogen, CN, NO₂, C1-C7 alkyl, C6-C12 aryl, C3-C12 heteroaryl containing one or more heteroatom(s) selected from N, O and S, 5-to 7-membered heterocycloalkyl or C1-C7 alkoxy; m represents an integer from 1 to 4; p represents an integer from 1 to 5; s represents an integer from 1 to 5; u represents an integer from 1 to 3; w represents an integer from 1 to 4; and the alkyl and alkoxy of R₁, R₃, R₄, R₅, R₆, R₂₁, R₂₂ and R₂₃ may be further substituted with one or more halogen, C3-C7 cycloalkyl or C1-C5 alkylamine.

A particularly preferred selenazole derivative activating PPAR represented by Chemical Formula I is one wherein: R₁ represents hydrogen, C1-C5 alkyl substituted with one or more fluorine, or fluorine; R₂ represents hydrogen, C1-C8 alkyl, 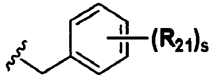 or 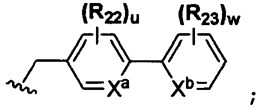 X^{a} and X^{b} independently represent CR or N; R represents hydrogen or C1-C8 alkyl; R₃ represents hydrogen, C1-C5 alkyl substituted or unsubstituted with halogen, or halogen; R₄ and R₅ independently represent hydrogen, C1-C5 alkyl substituted or unsubstituted with halogen; R₆ represents hydrogen, C1-C8 alkyl, halogen, allyl, C2-C7 alkenyl, a pharmaceutically acceptable organic salt, an alkali metal or an alkaline earth metal; and R₂₁, R₂₂ and R₂₃ independently represent hydrogen, halogen, CN, NO₂, C1-C7 alkyl substituted or unsubstituted with halogen, C6-C12 aryl, C3-C12 heteroaryl containing one or more heteroatom(s) selected from N, O and S, 5- to 7-membered heterocycloalkyl, or C1-C5 alkoxy substituted or unsubstituted with halogen.

In Chemical Formula I, R₁ may represent hydrogen, methyl, ethyl, n-propyl, i-propyl, n-butyl, t-butyl, n-pentyl, 2-ethylhexyl, fluoromethyl, difluoromethyl, trifluoromethyl, 2-fluoroethyl, pentafluoroethyl, fluorine, bromine, iodine or chlorine; R₂ may represent hydrogen or substituted or unsubstituted benzyl, phenylbenzyl or pyridylbenzyl, wherein the phenyl, pyridyl or benzyl of R₂ may be further substituted with fluorine, chlorine, methyl, ethyl, n-propyl, i-propyl, t-butyl, fluoromethyl, difluoromethyl, trifluoromethyl, 2-fluoroethyl, pentafluoroethyl, methoxy, ethoxy, propyloxy, n-butoxy, t-butoxy, fluoromethoxy, difluoromethoxy, trifluoromethoxy, 2-fluoroethoxy, pentafluoroethoxy, CN, NO₂, C6-C12 aryl or C3 -C12 heteroaryl containing one or more heteroatom (s) selected from N, O and S; R₃ may represent hydrogen, methyl, ethyl, n-propyl, i-propyl, n-butyl, t-butyl, n-pentyl, 2-ethylhexyl, fluoromethyl, difluoromethyl, trifluoromethyl, 2-fluoroethyl, pentafluoroethyl, fluorine, chlorine 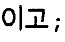 R₄ and R₅ may independently represent hydrogen, halogen, methyl, ethyl, n-propyl, i-propyl, n-butyl, t-butyl, n-pentyl, 2-ethylhexyl, fluoromethyl, difluoromethyl, trifluoromethyl, 2-fluoroethyl or pentafluoroethyl; and R₆ may represent hydrogen, methyl, ethyl, n-propyl, i-propyl, n-butyl, t-butyl, n-pentyl, 2-ethylhexyl, fluoromethyl, difluoromethyl, trifluoromethyl, 2-fluoroethyl, pentafluoroethyl, allyl, ethenyl, 2-propenyl, 2-butenyl, 3-butenyl, a pharmaceutically acceptable organic salt, Li⁺, Na⁺, K⁺, Ca²⁺ or Mg²⁺.

The novel compounds according to the present invention may be prepared by Schemes 1 to 5. In Scheme 1, A is O, NR, S or Se. In Scheme 2, A is NR. In Scheme 3, A is O.

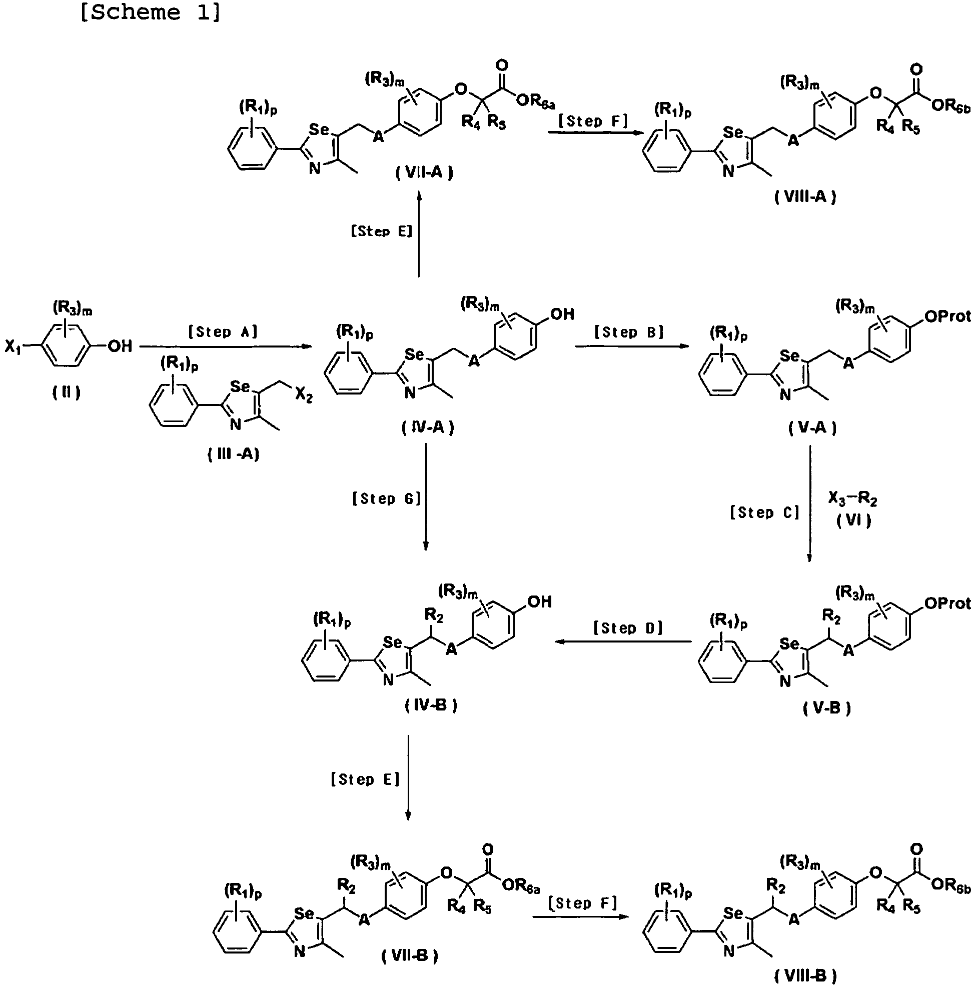

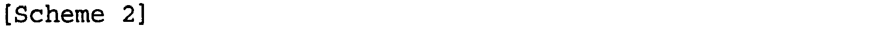

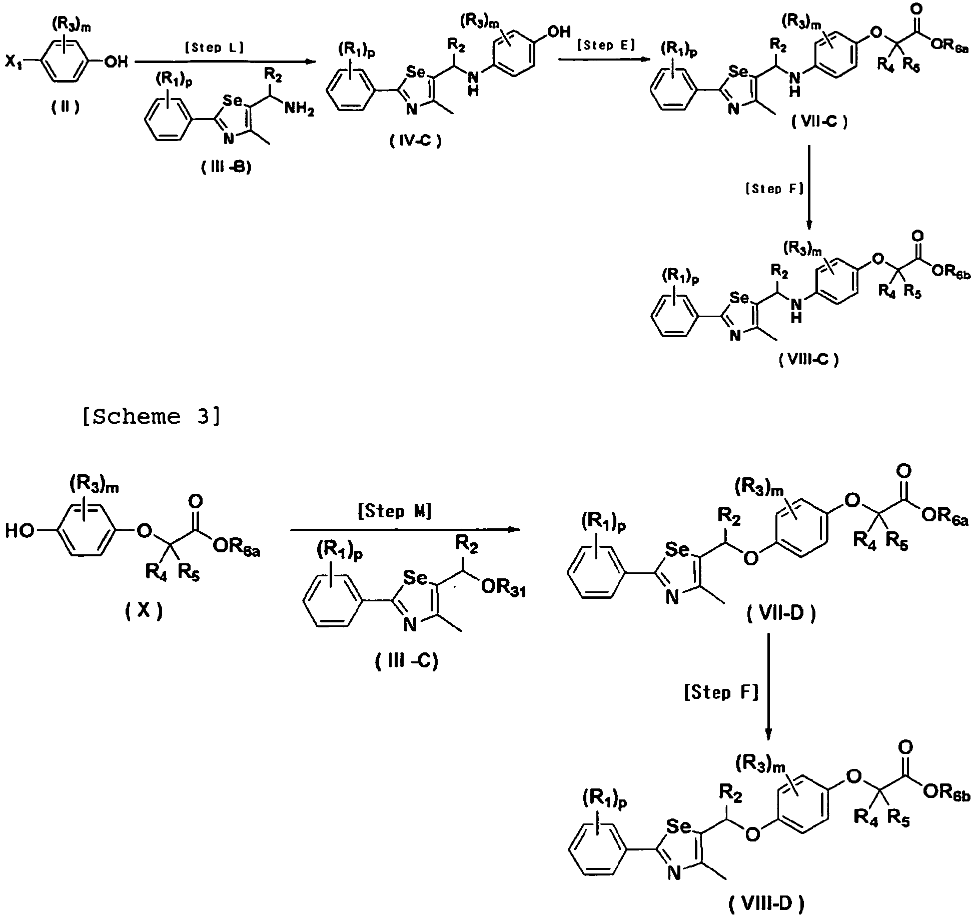

In Schemes 1 to 3, A represents O, NR, S or Se; R₁, R₂, R₃, m, p and s are the same as defined in Chemical Formula I; R₆ₐ represents C1-C8 alkyl or allyl; R_{6b} represents hydrogen, an alkali metal (Li⁺, Na⁺, K⁺), an alkaline earth metal (Ca²⁺, Mg²⁺) or a pharmaceutically acceptable organic salt; Prot represents a phenol protecting group selected from C1-C4 alkyl, allyl, alkylsilyl, alkylarylsilyl or tetrahydropyranyl; X₁ represents bromine or iodine; X₂ and X₃ independently represent chlorine, bromine, iodine or other leaving group suitable for nucleophilic substitution.

The compounds of Chemical Formulae III-A, III-B and III-C may be prepared by Scheme 4.

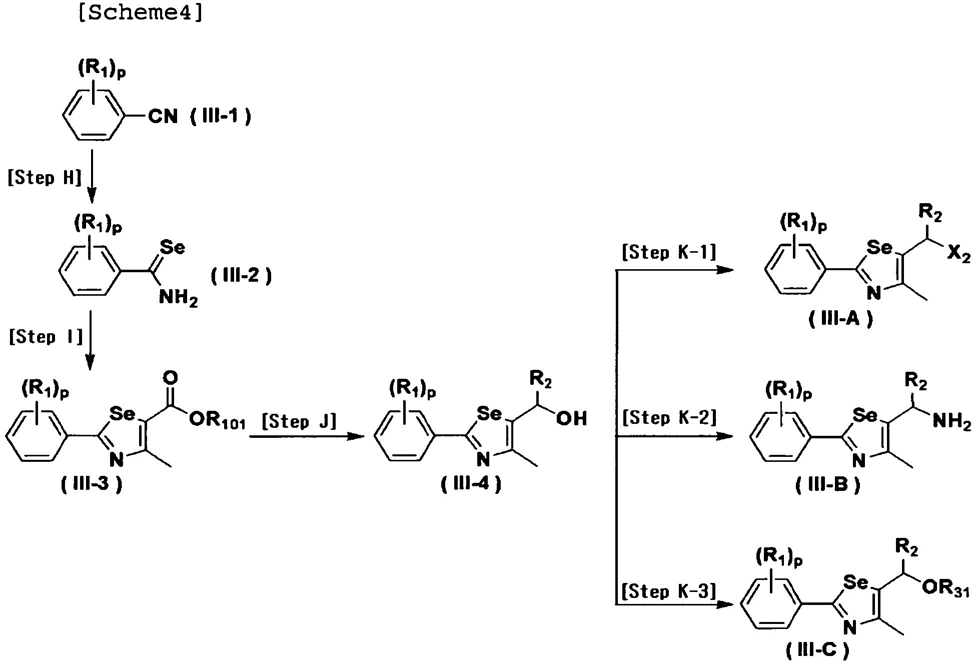

In Scheme 4, R₁, R₂ and p are the same as defined in Chemical Formula I; R₃₁ represents C1-C4 alkylsulfonyl, or C6-C12 arylsulfonyl substituted or unsubstituted with C1-C4 alkyl; R₁₀₁ represents C1-C4 alkyl; and X₂ represents chlorine, bromine, iodine or other leaving group suitable for nucleophilic substitution.

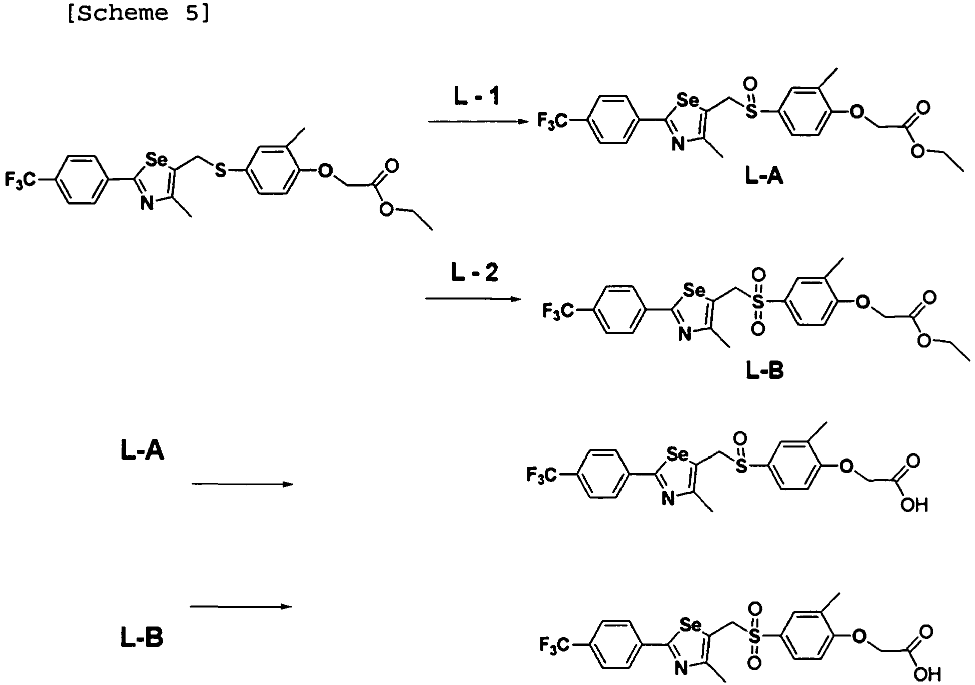

Hereinafter, the preparation method according to the present invention is described in detail.

### [Step A] Preparation of compound represented by Chemical Formula (IV-A)

In order to prepare the compound represented by Chemical Formula (IV-A), the phenol group of the compound represented by Chemical Formula (II) is protected with a Grignard reagent without a separation process. Subsequently, the resulting compound is reacted with an organometallic reagent and sulfur (S) or selenium (Se), and then with the compound represented by Chemical Formula (III-A). This step involves four-stage reactions that proceed at once.

Details are as follows.

### [Protection of phenol group using Grignard reagent]

As an anhydrous solvent, diethyl ether, tetrahydrofuran, hexane, heptane or a mixture of two or more of them is used. Among them, diethyl ether, tetrahydrofuran or a mixture solvent of diethyl ether and tetrahydrofuran is preferred. Particularly, a polar solvent is preferred. The most preferred is tetrahydrofuran.

The Grignard reagent may be methylmagnesium chloride, ethylmagnesium chloride, n-propylmagnesium chloride, isopropylmagnesium chloride, n-butylmagnesium chloride, sec-butylmagnesium chloride or alkylmagnesium bromide. Among them, the most preferred is isopropylmagnesium chloride ((CH₃)₂CHMgCl).

Reaction temperature may be different depending on the solvent used. Usually, the reaction is performed at -20 to 40°C, preferably at 0 °C to room temperature (25°C). Reaction time may be different depending on the reaction temperature and the solvent used. Usually, the reaction is performed for 10 to 60 minutes, preferably for 10 to 30 minutes.

[Halogen-lithium substitution and introduction of sulfur (S) or selenium (Se)]

In the halogen-lithium substitution, an organometallic reagent such as n-butyllithium, sec-butyllithium, tert-butyllithium, etc. may be used. Among them, tert-butyllithium is preferred.

Preferably, the sulfur (S) or selenium (Se) is in powder form with fine particles and is added directly or as dissolved in anhydrous tetrahydrofuran.

Reaction temperature may be different depending on the solvent used. Usually, the reaction is performed at-78 to 25°C. Preferably, the halogen-metal substitution is performed at -75°C, and the introduction of sulfur (S) or selenium (Se) is begun at -75°C and performed at room temperature (25°C). The halogen-metal substitution is performed for 10 to 30 minutes, and the introduction of sulfur (S) or selenium (Se) is performed for 30 to 120 minutes.

### [Addition of compound represented by Chemical Formula (III-A)]

The compound represented by Chemical Formula (III) is synthesized via Steps H and K. The halogen of the compound represented by Chemical Formula (III-A) may be chlorine, bromine or iodine. Among them, chlorine is preferred.

Reaction temperature may be different depending on the solvent used. Usually, the reaction is performed at-78 to 25°C, preferably at 0 to 10 °C. Reaction time is usually 10 to 120 minutes, preferably 10 to 60 minutes.

### [Step B] Preparation of compound represented by Chemical Formula (V-A)

In order to prepare the compound represented by Chemical Formula (V-A), the compound represented by Chemical Formula (IV-A) may be reacted with a compound commonly used to provide a phenol protecting group in the presence of a base.

The phenol protecting group may be C1-C4 alkyl, allyl, alkylsilyl such as trimethylsilyl, tert-butyldiphenylsilyl, triisopropylsilyl, tert-butyldimethylsilyl, etc., alkylarylsilyl, tetrahydropyranyl, or the like. Among them, tert-butyl, tetrahydropyranyl and silyl are preferred.

In this step, an aprotic polar solvent such as N,N-dimethylformamide, N,N-dimethylacetamide, dimethyl sulfoxide, acetonitrile, acetone, ethyl acetate, carbon tetrachloride, chloroform, dichloromethane, or the like may be used. Also, an ether such as tetrahydrofuran, dioxane, dimethoxyethane, diethylene glycol dimethyl ether, triethylene glycol dimethyl ether, or the like may beused. Also, an aromatic hydrocarbon such as benzene, toluene, xylene, or the like may be used. Among them, an aprotic polar solvent is preferred. The most preferred are N,N-dimethylformamide, chloroform and dichloromethane.

The base may be an amine-based based such as pyridine, triethylamine, imidazole, N,N-dimethylaminopyridine, or the like. The reaction for forming an alkyl or allyl ether protecting group is performed using sodium hydroxide, potassium hydroxide, sodium carbonate, potassium carbonate, or the like as the base. Among them, imidazole and potassium carbonate are preferred.

A tetrahydropyranyl protecting group is prepared by reacting 3,4-dihydro-2*H*-pyran with alkyl or allyl triphenylphosphonium bromide in the presence of a catalyst.

Reaction temperature may be different depending on the solvent used. Usually, the reaction is performed at -10 to 80 °C, preferably at 0 °C to room temperature (25°C). Reaction time may be different depending on the reaction temperature and the solvent used. Usually, the reaction is performed for 1 hour to 1 day, preferably for 4 hours or less.

### [Step C] Preparation of compound represented by Chemical Formula (V-B)

The compound represented by Chemical Formula (V-B) is prepared by treating the α-proton of the thio- or selenoether compound represented by Chemical Formula (V-A) with a strong base to prepare a nucleophile, and then reacting with various electrophiles.

In this step, as an anhydrous solvent, diethyl ether, tetrahydrofuran, hexane, heptane or a mixture of two or more of them is used. Among them, diethyl ether, tetrahydrofuran or a mixture solvent of diethyl ether and tetrahydrofuran is preferred.

For the extraction of α-proton, a strong base such as potassium tert-butoxide (t-BuOK), lithium diisopropylamide (LDA), n-butyllithium, sec-butyllithium, tert-butyllithium, or the like may be used. Among them, LDA is the most preferred.

The electrophile that reacts with the nucleophile may be a known compound which is easily available or can be easily prepared according to a known method. It may contain a highly reactive halogen, aldehyde or ketone group and is added directly or as dissolved in an anhydrous solvent.

Reaction temperature may be different depending on the solvent used. Usually, the reaction is performed at -78 to 25°C. Preferably, the extraction of α-proton using the strong base is performed at -75 °C. The electrophile is added at -75 °C and then the temperature is slowly raised to room temperature (25 °C). Reaction time may be different depending on stages. The extraction of α-proton using the strong base is performed for 10 to 30 minutes, and the reaction with the electrophile is performed for 30 to 90 minutes.

### [Step D] Preparation of compound represented by Chemical Formula (IV-B)

The compound represented by Chemical Formula (IV-B) is obtained by removing the phenol protecting group from the compound represented by Chemical Formula (V-B).

In this step, a polar solvent such as N,N-dimethylformamide, *N,N*-dimethylacetamide, dimethyl sulfoxide, acetonitrile, acetone, ethyl acetate, carbon tetrachloride, chloroform, dichloromethane, or the like may be used. As an ether, tetrahydrofuran, dioxane, dimethoxyethane, diethylene glycol dimethyl ether, or the like may be used. As an alcohol, methanol, ethanol, or the like may be used. As an aromatic hydrocarbon, benzene, toluene, xylene, or the like may be used. Among them, a polar solvent is preferred. The most preferred is tetrahydrofuran.

For the deprotection of the phenol protecting group, a Lewis acid such as trimethylsilyl iodide, sodium ethane thioalcohol, lithium iodide, aluminum halide, boron halide, trifluoroacetic acid, etc. is used for methyl, ethyl, tert-butyl, benzyl and allyl ether protecting groups, and a fluoride such as tetrabutylammonium fluoride (Bu₄N⁺F⁻), halogen acid (e.g., hydrofluoric acid, hydrochloric acid, bromic acid or iodic acid), potassium fluoride, etc. is used for silyl protecting groups such as trimethylsilyl, *tert*-butyldiphenylsilyl, triisopropylsilyl, *tert*-butyldimethylsilyl, etc. Among them, a fluoride is preferred for the removal of the silyl protecting group. More preferably, tetrabutylammonium fluoride may be used.

Reaction temperature may be different depending on the solvent used. Usually, the reaction is performed at 0 to 120 °C, preferably at 10°C to 25 °C. Reaction time may be different depending on the reaction temperature. Usually, the reaction is performed for 30 minutes to 1 day, preferably for 2 hours or less.

### [Step E] Preparation of compound represented by Chemical Formula (VII)

To obtain the compound represented by Chemical Formula (VII), the compound represented by Chemical Formula (IV) is reacted with halogen acetic acid alkyl ester or alkyl halogen acetic acid alkyl ester in the presence of a base.

The halogen acetic acid alkyl ester or the alkyl halogen acetic acid alkyl ester may be an easily available known compound. An unavailable alkyl halogen acetic acid alkyl ester may be prepared by bromination of alkyl acetic acid alkyl ester. The halogen may be chlorine, bromine, iodine, or the like.

In this step, an aqueous solvent such as N,N-dimethylformamide, N,N-dimethylacetamide, dimethyl sulfoxide, acetonitrile, acetone, ethanol and methanol or a mixture containing 1 to 10 % water may be usedasasolvent. Among them, acetone or dimethyl sulfoxide containing 1 to 5 % water is preferred the most.

The base may be either a weak base or a strong base without special limitation, as long as there is no negative effect on the reaction. The strong base may be an alkali metal hydride such as sodium hydride, lithium hydride, etc., an alkaline earthmetal hydride such as potassium hydride, etc., or an alkali metal hydroxide such as sodium hydroxide, potassium hydroxide, etc. Further, an alkali metal carbonate such as lithium carbonate, potassium carbonate, potassium bicarbonate, cesium carbonate, etc. may be used. Preferably, the base is an alkali metal carbonate, more preferably potassium carbonate.

Reaction temperature is not particularly limited as long as it is below the boiling point of the solvent. However, reaction at high temperature is not preferred because side reactions may occur. Usually, the reaction is performed at 0 to 90 °C. Reaction time may be different depending on reaction temperature. Usually, the reaction is performed for 30 minutes to 1 day, preferably for 30 to 120 minutes.

### [Step F-1] Preparation of Compound represented by Chemical Formula (VIII)

The compound represented by Chemical Formula (VIII) is prepared from carboxylic acid ester hydrolysis of the compound represented by Chemical Formula (VII) in a solution of water-soluble inorganic salt and alcohol, or from ester hydrolysis of the compound represented by Chemical Formula (VII) in a solution of 2.0 M lithium hydroxide in THF and water.

In this step, a water-miscible alcohol solvent such as methanol or ethanol is used.

Depending on the particular carboxylic acid alkali salt used, a 0.1 to 3 N aqueous solution of an alkali metal hydroxide such as lithium hydroxide, sodium hydroxide, potassium hydroxide, etc. is used as a base. Preferably, the acid used to obtain the compound represented by Chemical Formula (VIII) as a carboxylic acid may be acetic acid, sodium bisulfate (NaHS0₄) or 0.1 to 3 *N* HCl. Usually, 0. 5 M NaHSO₄ may be used to obtain the compound represented by Chemical Formula (VIII) as a carboxylic acid.

A low reaction temperature is preferred to prevent side reactions. Usually, the reaction is performed at 0 °C to room temperature. Reaction time may be different depending on reaction temperature. Usually, the reaction is performed for 10 minutes to 3 hours, preferably for 30 minutes to 1 hour. In case the reaction is performed in a solution of 2.0 M lithium hydroxide in THF and water, the reaction temperature is usually at 0 °C and the reaction time is preferably 1 to 2 hours.

### [Step F-2] Preparation of compound represented by Chemical Formula (VIII)

The compound represented by Chemical Formula (VIII) is prepared from allyl ester salt substitution of the compound represented by Chemical Formula (VII) in an organic solvent using a metal catalyst and an alkali metal salt or an alkaline earth metal salt of 2-ethylhexanoate.

In this step, an anhydrous organic solvent such as chloroform, dichloromethane, ethyl acetate, etc. is used.

The metal catalyst is tetrakis(triphenylphosphine)palladium (Pd(PPh₃)₄) and the metal catalyst may be used in an amount of 0.01 to 0.1 equivalent.

A low reaction temperature is preferred to prevent side reactions. Usually, the reaction is performed at 0 °C to room temperature. Reaction time may be different depending on reaction temperature. Usually, the reaction is performed for 10 minutes to 3 hours, preferably for 30 minutes to 1 hour.

The resulting salt compound is separated by centrifuge or using an ion exchange resin. The resulting metal salt compound represented by Chemical Formula (VIII) is easier to be separated than the salt compound obtained in Step F-1 (hydrolysis).

### [Step G] Preparation of compound represented by Chemical Formula (IV-B)

The compound represented by Chemical Formula (IV-B) is prepared by protecting the phenol group of the compound represented by Chemical Formula (IV-A) using a Grignard reagent without a separation process, treating the α-proton of the resulting thio- or selenoether with a strong base to prepare a nucleophile, and then reacting with various electrophiles. This step involves two-stage reactions that proceed at once.

Details are as follows.

### [Protection of phenol group using Grignard reagent]

In this step, diethyl ether, tetrahydrofuran, hexane, heptane or a mixture of two or more of them is used as an anhydrous solvent. Among them, diethyl ether, tetrahydrofuran or a mixture of diethyl ether and tetrahydrofuran is preferred the most. Especially, a polar solvent is preferred. The most preferred is tetrahydrofuran.

The Grignard reagent may be methylmagnesium chloride, ethylmagnesium chloride, n-propylmagnesium chloride, isopropylmagnesium chloride, n-butylmagnesium chloride, sec-butylmagnesium chloride or alkylmagnesium bromide. Among them, isopropylmagnesium chloride ((CH₃)₂CHMgCl) is preferred the most.

Reaction temperature may be different depending on the solvent used. Usually, the reaction is performed at -20 to 40°C, preferably at 0 °C to room temperature (25°C). Reaction time may be different depending on reaction temperature and the solvent used. Usually, the reaction is performed for 10 to 60 minutes, preferably for 10 to 30 minutes.

### [Extraction of α-proton and electrophilic addition]

The α-proton of the thio- or selenoether is treated with a strong base to prepare a nucleophile, which is then reacted with various electrophiles.

In this step, diethyl ether, tetrahydrofuran, hexane, heptane or a mixture of two or more of them is used as an anhydrous solvent. Among them, diethyl ether, tetrahydrofuran or a mixture of diethyl ether and tetrahydrofuran is preferred the most.

The strong base reagent used for the extraction of α-proton may be potassium tert-butoxide (t-BuOK), lithium diisopropylamide (LDA), n-butyllithium, sec-butyllithium, tert-butyllithium, or the like. Among them, LDA is preferred the most.

The electrophile that reacts with the nucleophile of the thio- or selenoether may be an easily available known compound or may be one that can be easily prepared by a known method. It may contain a highly reactive halogen, aldehyde or ketone group and is added directly or as dissolved in an anhydrous solvent.

Reaction temperature may be different depending on the solvent used. Usually, the reaction is performed at -78 to 25 °C. Preferably, the extraction of α-proton using the strong base is performed at -75 °C. The electrophile is added at -75 °C and then the temperature is slowly raised to room temperature (25 °C). Reaction time may be different depending on stages. The extraction of α-proton using the strong base is performed for 10 to 30 minutes, and the reaction with the electrophile is performed for 30 to 90 minutes.

### [Step H] Preparation of compound represented by Chemical Formula (III-2)

The compound represented by Chemical Formula (III-2) may be prepared by reducing metallic selenium with the strong reducing agent sodium borohydride in an alcohol solvent to prepare sodium hydrogen selenide, reacting it with the aryl nitrile compound represented by Chemical Formula (III-1) in a strong acid such as HCl under a reflux condition to prepare the selenocarbamate. In this step, an alcohol such as methanol and ethanol as well as a small amount of pyridine is used as a solvent. Preferably, sodium borohydride and selenium metal powder are used in equivalent amounts and 2 to 3 M HCl acid is used.

### [Step I] Preparation of compound represented by Chemical Formula (III-3)

The compound represented by Chemical Formula (III-3) is prepared by reacting the compound represented by Chemical Formula (III-2) with C1-C4 alkyl 2-chloroacetoacetate.

In this step, an alcohol such as methanol, ethanol, propanol, butanol, etc. or an ether such as ethyl ether, tetrahydrofuran, 1,4-dioxane, etc. may be used as a solvent. Among them, ethanol and tetrahydrofuran are preferred.

Reaction temperature may be different depending on the solvent used. Usually, the reaction is performed at 25 to 150 °C, preferably at 60 to 120°C. Reaction time may be different depending on the reaction temperature and the solvent used. Usually, the reaction is performed for 6 hours to 1 day, preferably for 16 hours or less.

### [Step J] Preparation of compound represented by Chemical Formula (III-4)

The alcohol compound represented by Chemical Formula (III-4) is prepared by reducing the ester compound represented by Chemical Formula (III-3) using a reducing agent.

The reducing agent used to reduce the ester may be an aluminum hydride reducing agent such as lithium aluminum hydride (LiAlH₄), diisobutylaluminum hydride (DIBAL-H), etc., or a borohydride reducing agent such as sodium borohydride, lithium borohydride, etc. Among them, the aluminum hydride reducing agent is preferred. The most preferred are LiAlH₄ and DIBAL-H.

In this step, diethyl ether, tetrahydrofuran, dichloromethane, or the like may be used as an anhydrous solvent. Dichloromethane is preferred the most.

Reaction time may be different depending on the solvent and the reducing agent used. Usually, the reaction is performed at -100 to 60 °C, preferably at -78 °C to 25 °C. Reaction time may be different depending on the reaction temperature and the solvent used. Usually, the reaction is performed for 30 minutes to 6 hours, preferably for 2 hours or less.

### [Step K] Preparation of compound represented by Chemical Formula (III)

The compound represented by Chemical Formula (III-A) may be prepared by halogenating the alcohol group of the compound represented by Chemical Formula (III-4). The compound represented by Chemical Formula (III-B) may be prepared from the compound represented by Chemical Formula (III-4) using NaN₃. And, the compound represented by Chemical Formula (III-C) may be prepared by introducing alkyl- or aryl-substituted sulfonyl chloride, preferably methanesulfonyl chloride or *p*-toluenesulfonyl chloride, at the hydroxyl group of the compound represented by Chemical Formula (III-4).

In the halogenations and the introduction of the methanesulfonyloxy or *p*-toluenesulfonyloxy group, *N,N*-dimethylformamide, diethyl ether, tetrahydrofuran, carbon tetrachloride, chloroform, dichloromethane, pyridine, or the like may be used as a solvent. Among them, dichloromethane is preferred the most for the halogenation, and pyridine is preferred the most for the introduction of the methanesulfonyloxy or *p*-toluenesulfonyloxy group.

The halogenation of alcohol may be carried out using triphenylphosphine (TPP) and *N*-chlorosuccinimide (NCS), triphenylphosphine and chlorine gas (Cl₂), triphenylphosphine and carbon tetrachloride (CCl₄), phosphorus pentachloride (PCl₅), thionyl chloride (SOCl₂) or methanesulfonyl chloride (MeSO₂Cl), or the like to introduce chlorine, using triphenylphosphine and *N*-bromosuccinimide (NBS), triphenylphosphine and bromine gas (BR3), triphenylphosphine and carbon tetrabromide (CBr₄), phosphorus pentabromide (PBr₅) or thionyl bromide (SOBr₂), or the like to introduce bromine, and using triphenylphosphine and *N-*iodosuccinimide, triphenylphosphine and solid iodine, triphenylphosphine and carbon tetraiodide (CI₄), or the like to introduce iodine or from halogen-iodine substitution of the chlorine or bromine compound represented by Chemical Formula (IV-A) in acetone. The introduction of the methanesulfonyloxy or *p*-toluenesulfonyloxy group may be performed by reacting with methanesulfonyl chloride or *p*-toluenesulfonyl chloride in pyridine solvent. The most preferred leaving group is chlorine or bromine, and the most preferred preparation method is one using triphenylphosphine and *N*-chlorosuccinimide or N-bromosuccinimide.

In this step, reaction temperature may be different depending on the preparation method and the solvent used. Usually, the reaction is performed at -10 to 40 °C, preferably at 10 to 25 °C. Reaction time may be different depending on the reaction temperature and the solvent used. Usually, the reaction is performed for 30 minutes to 1 day, preferably for 2 hours or less.

### [Step L] Preparation of compound represented by Chemical Formula (L-1) or (L-2)

The compound represented by Chemical Formula (L-1) may be prepared by dissolving the compound represented by Chemical Formula (VII) prepared in Step E in methylene chloride (CH₂Cl₂) and adding 1 equivalent of m-chloroperbenzoic acid (m-CPBA) while maintaining the reaction temperature at 0 to 5 °C. And, the compound represented by Chemical Formula (L-2) may be prepared by adding 2 equivalents of m-CPBA.

Thus prepared compound represented by Chemical Formula I is an important ligand of the PPAR protein. Since the compound has a chiral carbon, its stereoisomer exists. The present invention includes the selenazole derivative compound Chemical Formula I, a stereoisomer thereof, a solvate thereof and a salt thereof.

The selenazole derivative compound represented by Chemical Formula I according to the present invention or a pharmaceutically acceptable salt of the compound is useful as an activator of PPAR. Further, the selenazole derivative represented by Chemical Formula I according to the present invention, a hydrate thereof, a solvate thereof, a stereoisomer thereof and a pharmaceutically acceptable salt thereof are useful for a pharmaceutical composition, a functional food supplement composition, a functional drink composition, a food additive composition, a functional cosmetic composition or an animal feed composition for preventing or treating atherosclerosis, fatty liver or hyperlipemia, preventing or treating hypercholesterolemia, preventing or treating diabetes, preventing or treating obesity, strengthening muscle, improving endurance, improving memory, or preventing or treating dementia or Parkinson's disease, since they activate PPAR. The selenazole derivative represented by Chemical Formula I according to the present invention, a hydrate thereof, a solvate thereof, a stereoisomer thereof and a pharmaceutically acceptable salt thereof are useful for a functional cosmetic composition for preventing or improving obesity, preventing or improving fatty liver, strengthening muscle or improving endurance. The functional cosmetic composition may be prepared into ointment, lotion or cream and may be topically applied on the desired area of the body before and/or after exercise in order to strengthen muscles and improve endurance. Further, the selenazole derivative represented by Chemical Formula I according to the present invention, a hydrate thereof, a solvate thereof, a stereoisomer thereof and a pharmaceutically acceptable salt thereof may be prepared into ointment and topically applied in order to prevent or treat diabetes or diabetic foot ulcer.

The pharmaceutically acceptable salt may be a carboxylic acid salt of the selenazole derivative represented by Chemical Formula I or any other pharmaceutically acceptable organic salt (e.g., dicyclohexylamine or N-methyl-D-glucamine). A preferred inorganic salt includes an alkali metal salt and an alkaline earth metal salt of Li⁺, Na⁺, K⁺, Ca²⁺, Mg²⁺, or the like.

Of course, the quantity of the selenazole derivative represented by Chemical Formula I, a hydrate thereof, a solvate thereof, a stereoisomer thereof or a pharmaceutically acceptable salt thereof required to achieve a therapeutic effect depends on the particular compound, administration method, subject in need of treatment and disease desired to be treated, and may be determined as in other drugs. More preferably, an effective administration dose of the compound represented by Chemical Formula I is within 1 to 100 mg/kg (body weight)/day. Within the daily effective administration dose, it may be administered once or several times a day. Also, depending on the formulations, it may be administered orally or topically. A pharmaceutical composition for oral administration may be in any existing form, including, for example, tablet, powder, dry syrup, chewable tablet, granule, capsule, soft capsule, pill, drink, sublingual tablet, or the like. A tablet according to the present invention may be administered to a patient in any bioavailable mode or method, i.e. via an oral route. An adequate administration mode or method may be easily selected depending on the condition of the disease to be prevented or treated, progress of the disease, or other related situations. When the composition according to the present invention is a tablet, it may comprise one or more pharmaceutically acceptable excipient. The content and property of the excipient may be determined on the basis of solubility and chemical property of the selected tablet, selected administration route and standard pharmaceutical practice.

### [Description of Drawings]

The above and other obj ects, features and advantages of the present invention will become apparent from the following description of preferred embodiments given in conjunction with the accompanying drawing, in which:

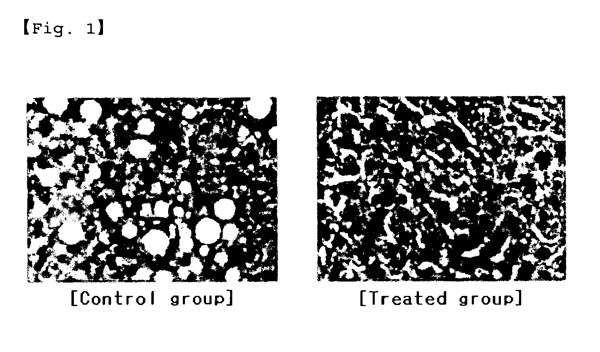

Fig. 1 shows a result of testing a fatty liver treating effect.

### [Mode for Invention]

The examples and experiments will now be described. The following examples and experiments are for illustrative purposes only and not intended to limit the scope of the present invention.

### [Examples]

### [Preparation Example 1] Preparation of Compound III-2a

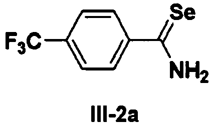

### [Step H]

Selenium powder (3.95 g, 50 mmol) was added to ethanol (50 mL) under nitrogen atmosphere. Then, sodium borohydride (2.02g, 53 mmol) wascautiouslyaddedslowlyfor30minutes (Hydrogen gas was produced.). To the resultant ethanolic sodium hydrogen selenide, 4-(trifluoromethyl)benzonitrile (11.9 g, 70 mmol) and pyridine (8 mL) were added. Then, 2 M hydrochloric acid (25 mL) was slowly added dropwise for 1. 5 hours while refluxing at 80 °C. After further stirring for about 30 minutes, the precipitated target compound was filtered and washed with hexane and water. Recrystallization using benzene solvent yielded Compound **III-2a** (15.1 g, yield: 91 %) as yellow solid.

¹H NMR (300 MHz, CDCl₃) δ 11.07 (b, 1H), 10.43 (b, 1H), 7.99 (d, 2H, J = 8.5 Hz), 7.77 (d, 2H, J = 8.3 Hz).

### [Preparation Example 2] Preparation of Compound III-3a

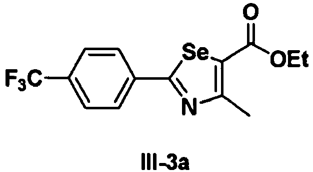

### [Step I]

Compound **III-2a** (2.52 g, 10.0 mmol) was dissolved at room temperature in tetrahydrofuran (35mL) and ethyl 2-chloroacetoacetate (1.22 mL, 10.0 mmol, 1.0 equivalent) was slowly added for 20 minutes. After completion of the addition, the mixture was further stirred at room temperature for 30 minutes and refluxed for 12 hours at 75 to 80 °C. After completion of the reaction, the temperature was lowered to room temperature and 50% sodium hydroxide aqueous solution (20 mL) was added. After stirring for 20 minutes, the organic layer was extracted with ethyl acetate and brine and dried with magnesium sulfate. After filtration, distillation under reduced pressure yielded Compound **III-3a** (3.33 g, yield: 95 %).

¹H NMR (300 MHz, CDCl₃) δ 8.02 (d, 2H, *J* = 8.1 Hz), 7.69 (d, 2H, *J* = 8.2 Hz), 3.88 (s, 3H), 2.79 (s, 3H).

### [Preparation Example 3] Preparation of Compound III-4a

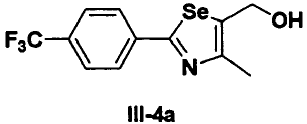

### [Step J]

The ethyl ester (Compound **III-3a,** 2.1 g, 6.0 mmol) obtained in

Preparation Example 2 was completely dissolved in anhydrous dichloromethane (100 mL) under nitrogen atmosphere and sufficiently cooled to -78 °C. Diisobutylaluminum hydride (DIBAL-H, 16.6 mL, 1.0 *M* hexane solution, 2.5 equivalents) was slowly added for 30 minutes. After performing reaction at the temperature for 30 minutes, reaction was further performed at -10 °C for 30 minutes. After completion of the reaction, reaction was terminated using ethyl acetate. After extraction with 10% sulfuric acid and ethyl acetate, the product was dried using magnesium sulfate. Filtration using a short silica gel column followed by distillation of the solvent under reduced pressure yielded Compound **III-4a** (1.74 g, yield: 94 %).

¹H NMR (300 MHz, CDCl₃) δ 7.96 (d, 2H, *J* = 8.1 Hz), 7.65 (d, 2H, *J* = 8.2 Hz), 4.88 (d, 2H, *J* = 5.3 Hz), 2.45 (s, 3H).

### [Preparation Example 4] Preparation of Compound III-A-1

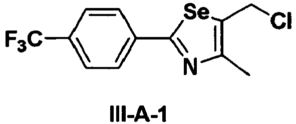

### [Step K-1]

Compound **III-4a** (1.13 g, 3.66 mmol) obtained in Preparation Example 3 was dissolved in anhydrous dichloromethane (30 mL). Then, triphenylphosphine (TPP, 1. 06 g, 4.03 mmol, 1.1 equivalents) was added and completely dissolved. At room temperature, *N*-chlorosuccinimide (717 mg, 4.03 mmol, 1.1 equivalents) was slowly added. After further stirring for 1 hour, the solvent was removed by distillation under reduced pressure. After precipitating triphenylphosphine oxide using hexane and ethyl acetate (5:1), filtration followed by distillation under reduced pressure yielded Compound **III-A-1** (1.07 g, yield: 90 %).

¹H NMR (300 MHz, CDCl₃) δ 7.95 (d, 2H, *J* = 8.2 Hz), 7.66 (d, 2H, *J* = 8.3 Hz), 4.84 (s, 2H), 2.48 (s, 3H).

### [Preparation Example 5] Preparation of Compound III-B-1

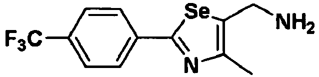

### [Step K-2]

Compound **III-4a** (352 mg, 1.1 equivalents) obtained in Preparation Example 3 was slowly dissolved in CCl₄-DMF (1:4, 5 mL). After adding PPh₃ (508 mg, 2.2 equivalents) and NaN₃ (78 mg, 1. 2 equivalents) dropwise, the temperature was slowly raised to 90 °C. When disappearance of the starting reagent was identified by TLC, the temperature was lowered to 25 °C. After further stirring for about 10 minutes, the reaction was terminated with distilled water (4 mL). After extraction with ethyl ether, the temperature was lowered to 0 °C. The crystallized triphenylphosphine oxide was removed by filtering. Flash silica column chromatography of the remaining product yielded Compound **III-B-1** (271 mg, yield: 85 %) (FABMS: 321[M+H]⁺).

### [Preparation Example 6] Preparation of Compound III-C-1

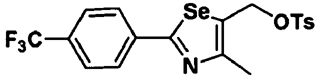

### [Step K-3]

Compound **III-4a** (352 mg, 1.1 equivalents) obtained in Preparation Example 3 was dissolved in methylene chloride (MC, 5 mL) and cooled to 0 °C. Then, after cautiously adding p-toluenesulfonyl chloride (*p*-TsCl, 190 mg, 1.0 equivalent) and Et₃N (1.5 equivalents), the mixture was further stirred. After completion of the reaction, concentration of the organic layer (MC solvent layer) was followed by flash silica column chromatography yielded Compound **III-C-1** (431 mg, yield: 91 %) (FABMS: 476[M+H]⁺).

### [Example 1] Preparation of Compound S1

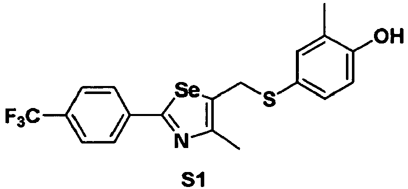

### [Step A]

4-Iodo-2-methylphenol (468 mg, 2 mmol) was dissolved in anhydrous tetrahydrofuran (20 mL) under nitrogen atmosphere and the temperature was maintained at 0 °C. After slowly adding isopropylmagnesium chloride (2 M, 1.5 mL), reaction was performed for 10 minutes. The mixture was sufficiently cooled to -78 °C and *tert*-butyllithium (2.00 mL, 1.7 M hexane solution, 1.0 equivalent) was slowly added. After stirring further for 10 minutes, solid sulfur (S, 64 mg, 2 mmol, 1.0 equivalent) was added at once at the same temperature. After performing reaction for 40 minutes until the temperature reached 15 °C, Compound III-A-1 (652mg, 2mmol, 1.0 equivalent) prepared in Preparation Example 4 was slowly added at the same temperature after being dissolved in anhydrous THF (10 mL). After further reacting for about 1 hour, the reaction was terminated with aqueous ammonium chloride solution. After extracting the organic solvent with ethyl acetate and brine, the organic layer was dried with magnesium sulfate. After filtration, followed by distillation under reduced pressure, purification of the residue by silica gel column chromatography yielded the target compound (705 mg, yield: 82 %).

¹H NMR (300 MHz, CDCl₃) δ 7.91 (d, 2H, *J* = 8.1 Hz), 7.63 (d, 2H, *J* = 8.0 Hz), 7.21 (m, 1H), 7.12 (m, 1H), 6.67 (d, 2H, *J* = 8.2 Hz), 4.15 (s, 2H), 2.19 (s, 3H), 2.17 (s, 3H).

### [Example 2] Preparation of Compound S2

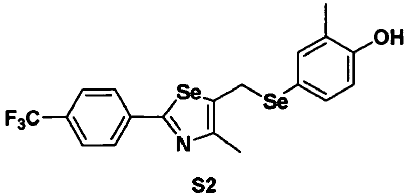

### [Step A]

4-Iodo-2-methylphenol (468 mg, 2 mmol) was dissolved in anhydrous tetrahydrofuran (20 mL) under nitrogen atmosphere and the temperature was maintained at 0 °C. After slowly adding isopropylmagnesium chloride (2 M, 1.5 mL), reaction was performed for 10 minutes. The mixture was sufficiently cooled to -78°C and tert-butyllithium (2.00 mL, 1.7 *M* hexane solution, 1.0 equivalent) was slowly added. After stirring further for 10 minutes, solid selenium (Se, 158 mg, 2 mmol, 1.0 equivalent) was added at once at the same temperature. After performing reaction for 40 minutes until the temperature reached 15 °C, Compound **III-A-1** (652 mg, 2 mmol, 1.0 equivalent) prepared in Preparation Example 4 was slowly added at the same temperature after being dissolved in anhydrous THF (10 mL). After further reacting for about 1 hour, the reaction was terminated with aqueous ammonium chloride solution. After extracting the organic solvent with ethyl acetate and brine, the organic layer was dried with magnesium sulfate. After filtration, followed by distillation under reduced pressure, purification of the residue by silica gel column chromatography yielded the target compound (773 mg, yield: 81 %).

¹H NMR (300 MHz, CDCl₃) δ 7.89 (d, 2H, *J* = 8.1 Hz), 7.63 (d, 2H, *J* = 8.2 Hz), 7.24 (m, 1H), 7.14 (m, 1H), 6.67 (d, 2H, *J* = 8.2 Hz), 4.17 (s, 2H), 2.18 (s, 3H), 2.13 (s, 3H).

### [Example 3] Preparation of Compound S3

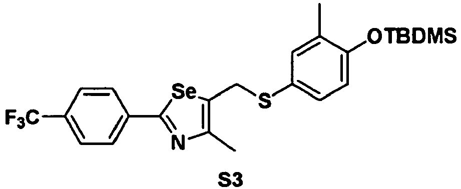

### [Step B]

Compound S1 (860mg, 2mmol) and imidazole (290mg, 2.0 equivalents) were completely dissolved in dimethylformamide (20 mL). After slowly adding tert-butyldimethylsilyl chloride (165 mg, 1.1 equivalents), reaction was performed at room temperature for 4 hours. After completion of the reaction, the organic solvent was extracted with aqueous ammonium chloride solution and ethyl acetate, and the organic layer was dried with magnesium sulfate. Purification using silica gel column followed by distillation under reduced pressure yielded the target compound (1053 mg, yield: 95 %). (FABMS: 558 [M+H]⁺).

### [Example 4] Preparation of Compound S4

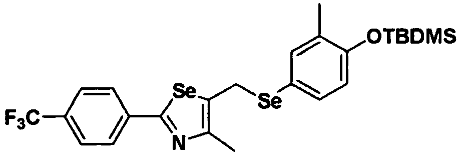

### [Step B]

Compound S2 (954mg, 2mmol) and imidazole (290mg, 2.0 equivalents) were completely dissolved in dimethylformamide (20 mL). After slowly adding tert-butyldimethylsilyl chloride (165 mg, 1.1 equivalents), reaction was performed at room temperature for 4 hours. After completion of the reaction, the organic solvent was extracted with aqueous ammonium chloride solution and ethyl acetate, and the organic layer was dried with magnesium sulfate. Purification using silica gel column followed by distillation under reduced pressure yielded the target compound (1099 mg, yield: 93 %). (FABMS: 606[M+H]⁺).

### [Example 5] Preparation of Compound S5

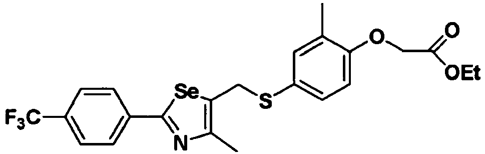

### [Step E]

Compound S1 (430 mg, 1 mmol), acetone (10 mL) containing 5% water, and potassium carbonate (346 mg, 2.5 mmol, 2.5 equivalents) were mixed well at room temperature. After adding bromoacetic acid ethyl ester (134 µL, 1.2 mmol, 1.2 equivalents), the mixture was vigorously stirred for 4 hours. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (480 mg, yield: 93 %).

¹H NMR (300 MHz, CDCl₃) δ 7.90 (d, 2H, *J* = 8.1 Hz), 7.64 (d, 2H, *J* = 8.2 Hz), 7.25 (m, 1H), 7.14 (m, 1H), 6.67 (d, 2H, *J* = 8.2 Hz), 4.61 (s, 2H), 4.23 (m, 2H), 4.16 (s, 2H), 2.24 (s, 3H), 2.21 (s, 3H), 1.28 (t, 3H, *J* = 3.7 Hz).

### [Example 6] Preparation of Compound S6

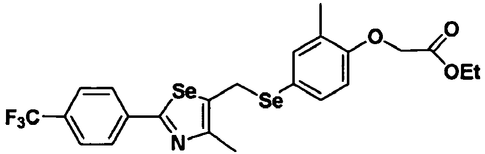

### [Step E]

Compound S2 (477 mg, 1 mmol), acetone (10 mL) containing 5% water, and potassium carbonate (346 mg, 2.5 mmol, 2.5 equivalents) were mixed well at room temperature. After adding bromoacetic acid ethyl ester (134 µL, 1.2 mmol, 1. 2 equivalents), the mixture was vigorously stirred for 4 hours. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (523 mg, yield: 93 %).

¹H NMR (300 MHz, CDCl₃) δ 7.90 (d, 2H, *J* = 8.1 Hz), 7.64 (d, 2H, *J* = 8.2 Hz), 7.27 (m, 1H), 7.20 (m, 1H), 6.68 (d, 2H, *J* = 8.2 Hz), 4.61 (s, 2H), 4.23 (m, 2H), 4.19 (s, 2H), 2.23 (s, 3H), 2.15 (s, 3H), 1.27 (t, 3H, *J* = 3.7 Hz).

### [Example 7] Preparation of Compound S7

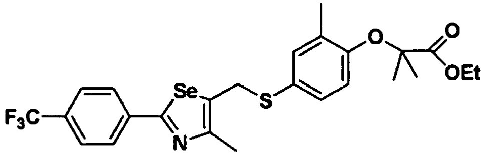

### [Step E]

Compound S1 (430 mg, 1 mmol), acetone (10 mL) containing 5% water, and potassium carbonate (346 mg, 2.5 mmol, 2.5 equivalents) were mixed well at room temperature. After adding ethyl-2-bromo-2-methylpropanoate (210 µL, 1.2 mmol, 1.2 equivalents), the mixture was vigorously stirred for 4 hours, while supplementing acetone and heating to 60 to 90 °C. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (326 mg, yield: 60 %). (FABMS: 558 [M+H]⁺).

### [Example 8] Preparation of Compound S8

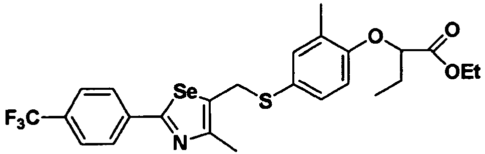

### [Step E]

Compound **S1** (430 mg, 1 mmol), acetone (10 mL) containing 5% water, and potassium carbonate (346 mg, 2.5 mmol, 2.5 equivalents) were mixed well at room temperature. After adding ethyl-2-bromobutylate (146 µL, 1.2 mmol, 1.2 equivalents), the mixture was vigorously stirred for 4 hours, while supplementing acetone and heating to 60 to 90 °C. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (451 mg, yield: 83 %). (FABMS: 558 [M+H]⁺).

### [Example 9] Preparation of Compound S9

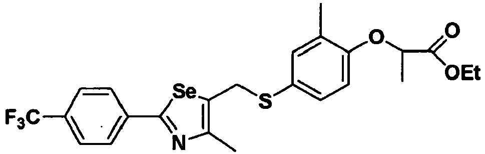

### [Step E]

Compound S1 (430 mg, 1 mmol), acetone (10 mL) containing 5% water, and potassium carbonate (346 mg, 2.5 mmol, 2.5 equivalents) were mixed well at room temperature. After adding ethyl-2-bromopropionate (155 µL, 1.2 mmol, 1.2 equivalents), the mixture was vigorously stirred for 4 hours, while supplementing acetone and heating to 60 to 90 °C. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (429 mg, yield: 81 %). (FABMS: 544 [M+H]⁺).

### [Example 10] Preparation of Compound S10

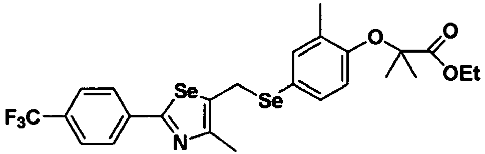

### [Step E]

Compound **S2** (478 mg, 1 mmol), acetone (10 mL) containing 5% water, and potassium carbonate (346 mg, 2.5 mmol, 2.5 equivalents) were mixed well at room temperature. After adding ethyl-2-bromo-2-methylpropanoate (210 µL, 1.2 mmol, 1.2 equivalents), the mixture was vigorously stirred for 4 hours, while supplementing acetone and heating to 60 to 90 °C. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (349 mg, yield: 59 %). (FABMS: 606 [M+H]⁺).

### [Example 11] Preparation of Compound S11

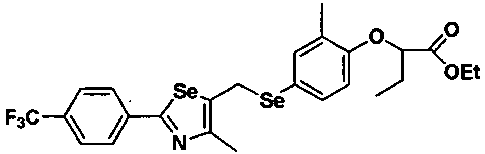

### [Step E]

Compound **S2** (478 mg, 1 mmol), acetone (10 mL) containing 5% water, and potassium carbonate (346 mg, 2.5 mmol, 2. 5 equivalents) were mixed well at room temperature. After adding ethyl-2-bromobutylate (146 µL, 1.2 mmol, 1.2 equivalents), the mixture was vigorously stirred for 4 hours, while supplementing acetone and heating to 60 to 90 °C. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (490 mg, yield: 83 %). (FABMS: 606 [M+H]⁺).

### [Example 12] Preparation of Compound S12

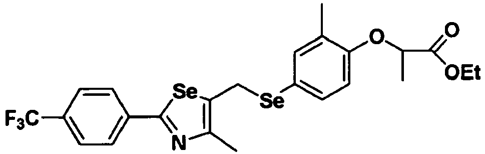

### [Step E]

Compound S2 (478 mg, 1 mmol), acetone (10 mL) containing 5% water, and potassium carbonate (346 mg, 2.5 mmol, 2.5 equivalents) were mixed well at room temperature. After adding ethyl-2-bromopropionate (155 µL, 1.2 mmol, 1.2 equivalents), the mixture was vigorously stirred for 4 hours, while supplementing acetone and heating to 60 to 90 °C. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (462 mg, yield: 80 %). (FABMS: 592 [M+H]⁺).

### [Example 13] Preparation of Compound S13

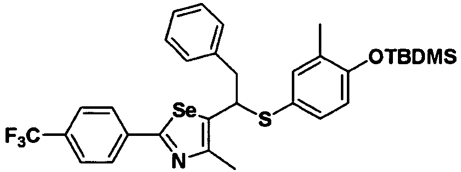

### [Step C]

Compound **S3** (544 mg, 1 mmol) was dissolved in anhydrous tetrahydrofuran (10 mL) and the temperature was lowered to -78 °C. Then, lithium diisopropylamide (LDA, 1.8 mL, 1.8 *M*, 2.0 equivalents) was slowly added. After adding benzyl bromide (137 µL, 1.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (526 mg, yield: 83 %). (FABMS: 648 (M+H]⁺).

### [Example 14] Preparation of Compound S14

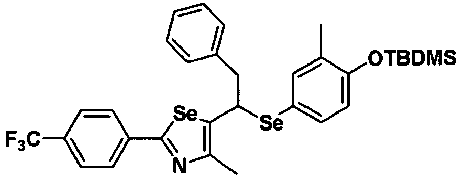

### [Step C]

Compound **S4** (591 mg, 1 mmol) was dissolved in anhydrous tetrahydrofuran (10 mL) and the temperature was lowered to -78 °C. Then, lithium diisopropylamide (LDA, 1.8 mL, 1.8 *M*, 2.0 equivalents) was slowly added. After adding benzyl bromide (137 µL, 1.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (538 mg, yield: 79 %). (FABMS: 694 [M+H]⁺).

### [Example 15] Preparation of Compound S15

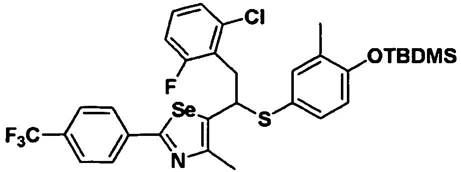

### [Step C]

Compound S3 (699 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 2-chloro-5-fluorobenzyl bromide (270 µL, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (531 mg, yield: 76 %). (FABMS: 700 [M+H]⁺).

### [Example 16] Preparation of Compound S16

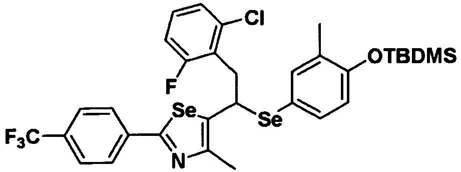

### [Step C]

Compound **S4** (746 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 2-chloro-5-fluorobenzyl bromide (270 µL, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (552 mg, yield: 74 %). (FABMS: 746 [M+H]⁺).

### [Example 17] Preparation of Compound S17

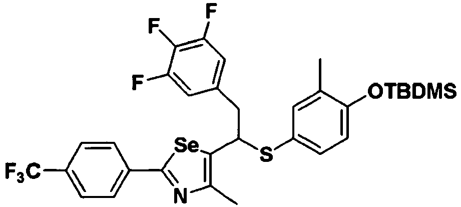

### [Step C]

Compound **S3** (700 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 3,4,5-trifluorobenzyl bromide (282 µL, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (525 mg, yield: 75 %). (FABMS: 702 [M+H]⁺).

### [Example 18] Preparation of Compound S18

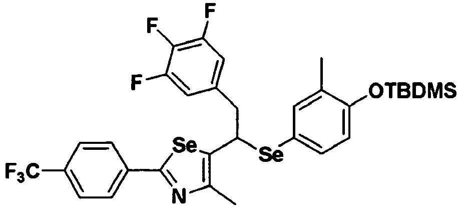

### [Step C]

Compound **S4** (747 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 3,4,5-trifluorobenzyl bromide (282 µL, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (523 mg, yield: 70 %). (FABMS: 750 [M+H]⁺).

### [Example 19] Preparation of Compound S19

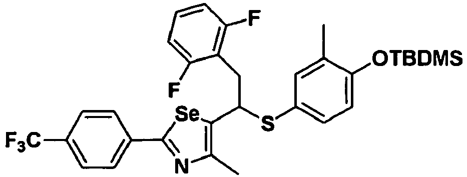

### [Step C]

Compound **S3** (682 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 2,5-difluorobenzyl bromide (259 µL, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (518 mg, yield: 76 %). (FABMS: 684 [M+H]⁺).

### [Example 20] Preparation of Compound S20

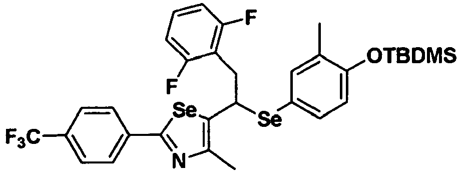

### [Step C]

Compound **S4** (724 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 2,5-difluorobenzyl bromide (259 µL, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (514 mg, yield: 71 %). (FABMS: 732 [M+H]⁺).

### [Example 21] Preparation of Compound S21

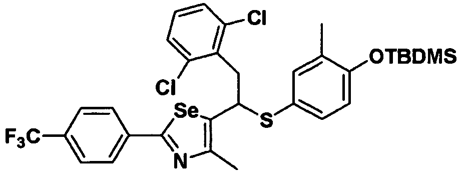

### [Step C]

Compound **S3** (715 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 2,5-dichlorobenzyl bromide (300 µL, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (529 mg, yield: 74 %). (FABMS: 716 [M+H]⁺).

### [Example 22] Preparation of Compound S22

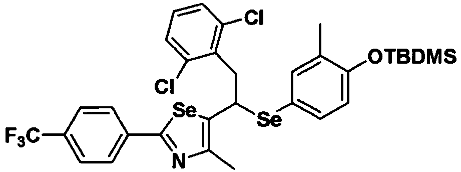

### [Step C]

Compound **S4** (762 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 2,5-dichlorobenzyl bromide (300 µL, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (541 mg, yield: 71 %). (FABMS: 762 [M+H]⁺).

### [Example 23] Preparation of Compound S23

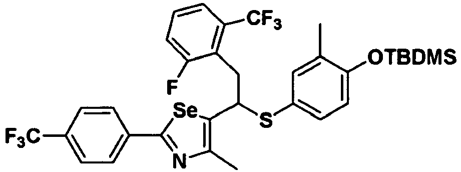

### [Step C]

Compound **S3** (732 mg, 1 mmol) was dissolved in anhydrous tetrahydrofuran (20 mL) and the temperature was lowered to -78 °C. Then, lithium diisopropylamide (LDA, 1.8 mL, 1.8 *M*, 2.0 equivalents) was slowly added. After adding 2-fluoro-5-trifluoromethylbenzyl bromide (514 mg, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (534 mg, yield: 73 %). (FABMS: 734 [M+H] ⁺).

### [Example 24] Preparation of Compound S24

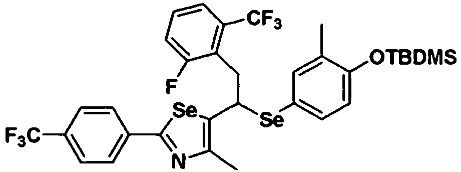

### [Step C]

Compound S4 (779 mg, 1 mmol) was dissolved in anhydrous tetrahydrofuran (20 mL) and the temperature was lowered to -78 °C. Then, lithium diisopropylamide (LDA, 1.8 mL, 1.8 M, 2.0 equivalents) was slowly added. After adding 2-fluoro-5-trifluoromethylbenzyl bromide (514 mg, 2.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (545 mg, yield: 70 %). (FABMS: 782 [M+H]⁺) .

### [Example 25] Preparation of Compound S25

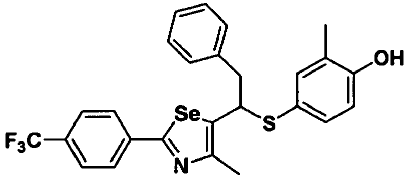

### [Step G]

Compound S1 (430 mg, 1 mmol) was dissolved in anhydrous tetrahydrofuran (10 mL) under nitrogen atmosphere and the temperature was maintained at 0 °C. After slowly adding isopropylmagnesium chloride (2 M, 1 mL), reaction was performed for 10 minutes. After sufficiently cooling to -78 °C, lithium diisopropylamide (LDA, 1.8 mL, 1.8 M, 2.0 equivalents) was slowly added. After adding benzyl bromide (137 µL, 1.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (426 mg, yield: 80 %). (FABMS: 534 [M+H]⁺).

### [Example 26] Preparation of Compound S26

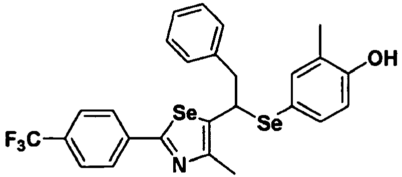

### [Step G]

Compound S2 (478 mg, 1 mmol) was dissolved in anhydrous tetrahydrofuran (10 mL) under nitrogen atmosphere and the temperature was maintained at 0 °C. After slowly adding isopropylmagnesium chloride (2 M, 1 mL), reaction was performed for 10 minutes. After sufficiently cooling to -78 °C, lithium diisopropylamide (LDA, 1.8 mL, 1.8 M, 2.0 equivalents) was slowly added. After adding benzyl bromide (137 µL, 1.0 mmol), the temperature was slowly raised to room temperature. After further reacting for 30 minutes, the reaction was terminated with aqueous ammonium chloride solution. The organic solvent was extracted with ethyl acetate and brine, and the organic layer was dried with magnesium sulfate. After filtration, followed by distillation under reduced pressure, purification of the residue by silica gel column chromatography yielded the target compound (457 mg, yield: 78 %). (FABMS: 582 [M+H]⁺).

### [Examples 27 to 36]

Compounds S27 to S46 listed in Table 1 were prepared according to the procedure of Examples 25 and 26. MS analysis result is given in the table.

**[Table 1]**

| | | | | | | |
|---|---|---|---|---|---|---|
| 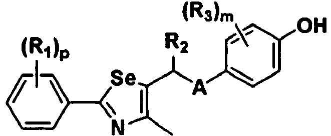 | | | | | | |

| **Exampl**e | **Compound No.** | 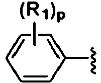 | R₂ | 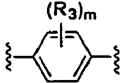 | A | FABMS: |
|---|---|---|---|---|---|---|
| 27 | S27 | 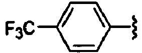 | 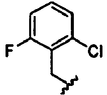 | 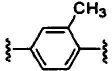 | S | (586[M+H]⁺). |
| 28 | S28 | 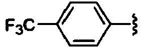 | 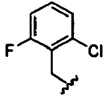 | 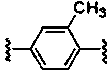 | Se | (633[M+H]⁺). |
| 29 | S29 | 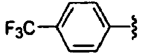 | 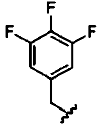 | 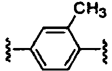 | S | (588[M+H]⁺). |
| 30 | S30 | 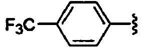 | 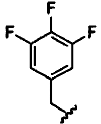 | 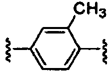 | Se | (636[M+H]⁺). |
| 31 | S31 | 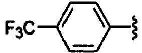 | 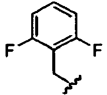 | 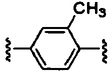 | S | (570[M+H]⁺). |
| 32 | S32 | 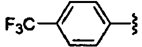 | 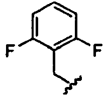 | 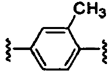 | Se | (618[M+H]⁺). |
| 33 | S33 | 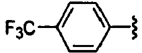 | 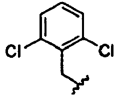 | 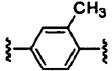 | S | (602[M+H]⁺). |
| 34 | S34 | 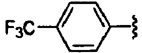 | 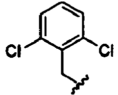 | 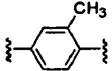 | Se | (650[M+H]⁺). |
| 35 | S35 | 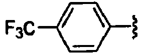 | 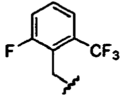 | 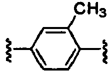 | S | (620[M+H]⁺). |
| 36 | S36 | 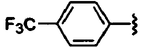 | 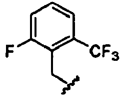 | 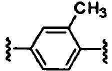 | Se | (668[M+H]⁺). |
| 37 | S37 | 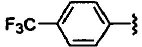 | 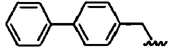 | 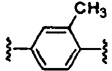 | S | (610[M+H]⁺). |
| 38 | S38 | 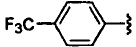 | 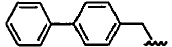 | 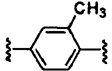 | Se | (658[M+H]⁺). |
| 39 | S39 | 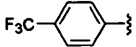 | 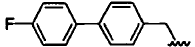 | 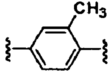 | S | (628[M+H]⁺). |
| 40 | S40 | 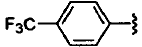 | 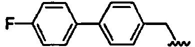 | 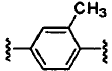 | Se | (676[M+H]⁺). |
| 41 | S41 | 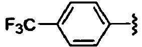 | 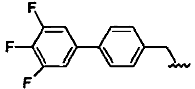 | 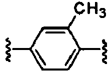 | S | (664[M+H]⁺). |
| 42 | S42 | 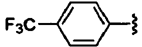 | 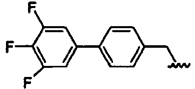 | 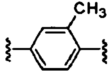 | Se | (712[M+H]⁺). |
| 43 | S43 | 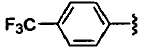 | 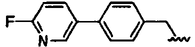 | 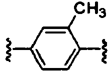 | S | (629[M+H]⁺). |
| 44 | S44 | 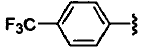 | 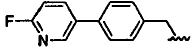 | 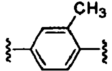 | Se | (677[M+H]⁺). |
| 45 | S45 | 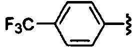 | 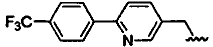 | 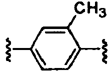 | S | (665[M+H]⁺). |
| 46 | S46 | 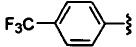 | 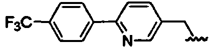 | 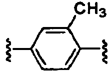 | Se | (713[M+H]⁺). |

### [Example 47] Preparation of Compound S47

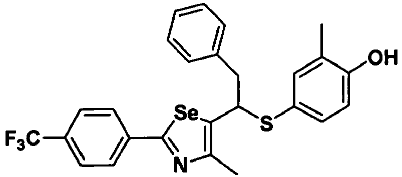

### [Step D]

Compound S13 (646 mg, 1 mmol) was completely dissolved in tetrahydrofuran (10 mL). Then, tetrabutylammonium fluoride (TBAF, 2.5 mL, 1 M tetrahydrofuran solution, 2.5 equivalents) was slowly added at room temperature. After reacting for 30 minutes, the organic solvent was extracted with aqueous ammonium chloride solution and ethyl acetate, and the organic layer was dried with magnesium sulfate. After filtration, followed by distillation under reduced pressure, purification of the residue by silica gel column chromatography yielded the target compound (479 mg, yield: 92 %). (FABMS: 534 [M+H]⁺).

### [Example 48] Preparation of Compound S48

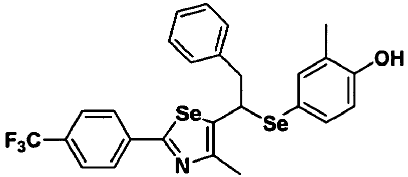

### [Step D]

Compound S14 (693 mg, 1 mmol) was completely dissolved in tetrahydrofuran (10 mL). Then, tetrabutylammonium fluoride (TBAF, 2.5 mL, 1 M tetrahydrofuran solution, 2.5 equivalents) was slowly added at room temperature. After reacting for 30 minutes, the organic solvent was extracted with aqueous ammonium chloride solution and ethyl acetate, and the organic layer was dried with magnesium sulfate. After filtration, followed by distillation under reduced pressure, purification of the residue by silica gel column chromatography yielded the target compound (521 mg, yield: 90 %). (FABMS: 582 [M+H]⁺).

Compounds S27 to S46 can be prepared according to the procedure of Examples 47 and 48.

### [Example 49] Preparation of Compound S49

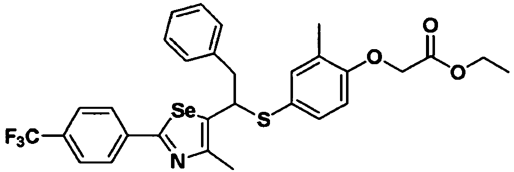

### [Step E]

Compound S25 (532 mg, 1 mmol) and acetone (10 mL) containing 5% water, and potassium carbonate (346 mg, 2.5 mmol, 2.5 equivalents) were mixed well at room temperature. After adding bromoacetic acid ethyl ester (134 µL, 1.2 mmol, 1.2 equivalents), the mixture was vigorously stirred for 4 hours. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (575 mg, yield: 93 %). (FABMS: 620 M+H]⁺).

### [Example 50] Preparation of Compound S50

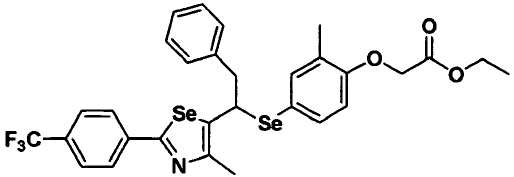

### [Step E]

Compound S26 (579 mg, 1 mmol) and acetone (10 mL) containing 5% water, and potassium carbonate (346 mg, 2.5 mmol, 2.5 equivalents) were mixed well at room temperature. After adding bromoacetic acid ethyl ester (134 µL, 1.2 mmol, 1.2 equivalents), the mixture was vigorously stirred for 4 hours. After completion of the reaction, the organic solvent was extracted with brine and ethyl acetate, and the organic layer was dried with magnesium sulfate. After filtration, followed by distillation under reduced pressure, purification of the residue by silica gel column chromatography using hexane/ethyl acetate (v/v = 5:1) yielded the target compound (605 mg, yield: 91 %). (FABMS: 668 [M+H]⁺).

### [Examples 51 to 136]

Compounds S51 to S136 listed in Table 2 were prepared according to the procedure of Examples 49 and 50. MS analysis result is given in the table.

**[Table 2]**

| | | | | | | | | | |
|---|---|---|---|---|---|---|---|---|---|
| 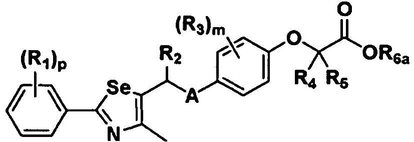 | | | | | | | | | |

| Example | Compound No. | 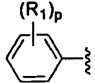 | R₂ | 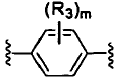 | A | R₄ | R₅ | R₆ₐ | FABMS: |
|---|---|---|---|---|---|---|---|---|---|
| 51 | S51 | 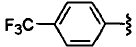 | 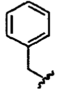 | 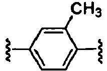 | S | CH₃ | CH₃ | CH₂CH₃ | (648[M+H]⁺) |
| 52 | S52 | 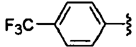 | 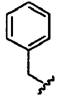 | 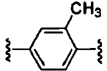 | Se | CH₃ | CH₃ | CH₂CH₃ | (696[M+H]⁺) |
| 53 | S53 | 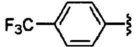 | 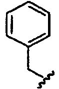 | 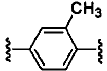 | S | H | CH₂CH₃ | CH₂CH₃ | (648[M+H]⁺) |
| 54 | S54 | 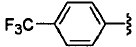 | 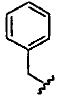 | 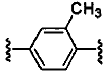 | Se | H | CH₂CH₃ | CH₂CH₃ | (696[M+H]⁺) |
| 55 | S55 | 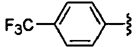 | 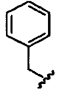 | 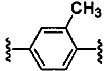 | S | H | CH₃ | CH₂CH₃ | (634[M+H]⁺) |
| 56 | S56 | 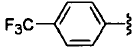 | 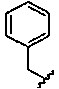 | 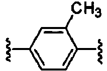 | Se | H | CH₃ | CH₂CH₃ | (682[M+H]⁺) |
| 57 | S57 | 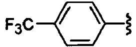 | 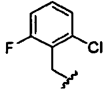 | 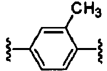 | S | H | H | CH₂CH₃ | (672[M+H]⁺) |
| 58 | S58 | 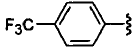 | 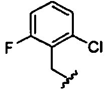 | 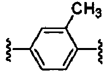 | Se | H | H | CH₂CH₃ | (718[M+H]⁺) |
| 59 | S59 | 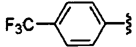 | 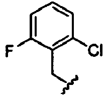 | 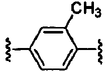 | S | CH₃ | CH₃ | CH₂CH₃ | (700[M+H]⁺) |
| 60 | S60 | 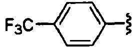 | 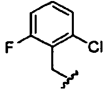 | 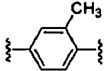 | Se | CH₃ | CH₃ | CH₂CH₃ | (748[M+H]⁺) |
| 61 | S61 | 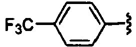 | 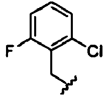 | 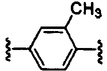 | S | H | CH₂CH₃ | CH₂CH₃ | (700[M+H]⁺) |
| 62 | S62 | 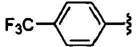 | 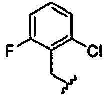 | 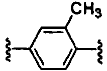 | Se | H | CH₂CH₃ | CH₂CH₃ | (748[M+H]⁺) |
| 63 | S63 | 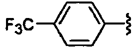 | 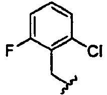 | 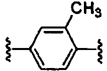 | S | H | CH₃ | CH₂CH₃ | (686[M+H]⁺) |
| 64 | S64 | 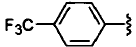 | 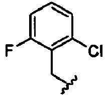 | 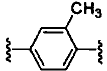 | Se | H | CH₃ | CH₂CH₃ | (734[M+H]⁺) |
| 65 | S65 | 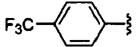 | 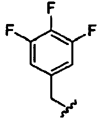 | 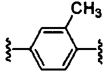 | S | H | H | CH₂CH₃ | (674[M+H]⁺) |
| 66 | S66 | 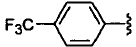 | 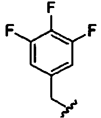 | 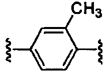 | Se | H | H | CH₂CH₃ | (722[M+H]⁺) |
| 67 | S67 | 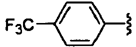 | 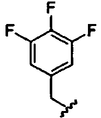 | 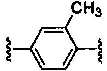 | S | CH₃ | CH₃ | CH₂CH₃ | (702[M+H]⁺) |
| 68 | S68 | 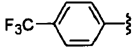 | 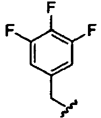 | 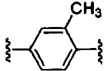 | Se | CH₃ | CH₃ | CH₂CH₃ | (750[M+H]⁺) |
| 69 | S69 | 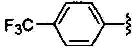 | 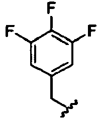 | 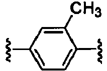 | S | H | CH₂CH₃ | CH₂CH₃ | (702[M+H)⁺) |
| 70 | S70 | 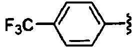 | 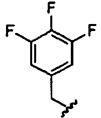 | 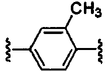 | Se | H | CH₂CH₃ | CH₂CH₃ | (750[M+H]⁺) |
| 71 | S71 | 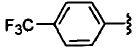 | 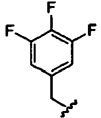 | 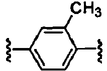 | S | H | CH₃ | CH₂CH₃ | (688[M+H]⁺) |
| 72 | S72 | 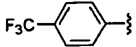 | 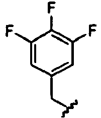 | 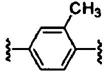 | Se | H | CH₃ | CH₂CH₃ | (736[M+H]⁺) |
| 73 | S73 | 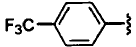 | 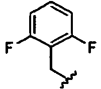 | 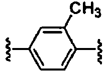 | S | H | H | CH₂CH₃ | (656[M+H]⁺) |
| 74 | S74 | 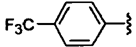 | 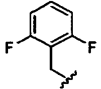 | 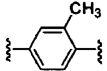 | Se | H | H | CH₂CH₃ | (704[M+H]⁺) |
| 75 | S75 | 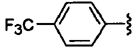 | 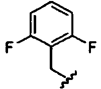 | 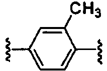 | S | CH₃ | CH₃ | CH₂CH₃ | (684[M+H]⁺) |
| 76 | S76 | 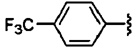 | 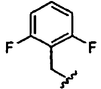 | 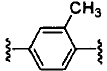 | Se | CH₃ | CH₃ | CH₂CH₃ | (732[M+H]⁺) |
| 77 | S77 | 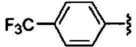 | 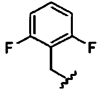 | 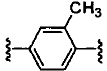 | S | H | CH₂CH₃ | CH₂CH₃ | (684[M+H]⁺) |
| 78 | S78 | 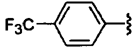 | 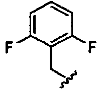 | 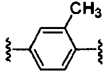 | Se | H | CH₂CH₃ | CH₂CH₃ | (732[M+H]⁺) |
| 79 | S79 | 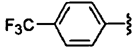 | 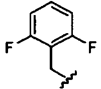 | 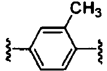 | S | H | CH₃ | CH₂CH₃ | (670[M+H]⁺) |
| 80 | S80 | 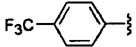 | 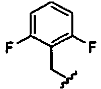 | 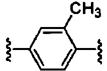 | Se | H | CH₃ | CH₂CH₃ | (718[M+H]⁺) |
| 81 | S81 | 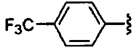 | 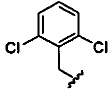 | 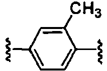 | S | H | H | CH₂CH₃ | (688[M+H]⁺) |
| 82 | S82 | 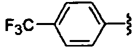 | 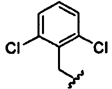 | 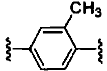 | Se | H | H | CH₂CH₃ | (736[M+H]⁺) |
| 83 | S83 | 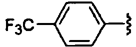 | 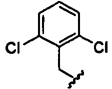 | 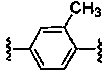 | S | CH₃ | CH₃ | CH₂CH₃ | (716[M+H]⁺) |
| 84 | S84 | 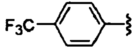 | 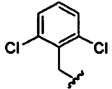 | 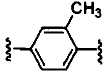 | Se | CH₃ | CH₃ | CH₂CH₃ | (764[M+H]⁺) |
| 85 | S85 | 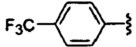 | 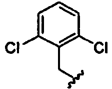 | 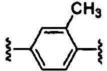 | S | H | CH₂CH₃ | CH₂CH₃ | (716[M+H]⁺) |
| 86 | S86 | 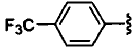 | 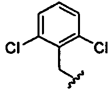 | 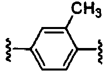 | Se | H | CH₂CH₃ | CH₂CH₃ | (764[M+H]⁺) |
| 87 | S87 | 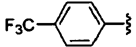 | 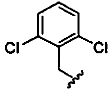 | 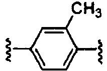 | S | H | CH₃ | CH₂CH₃ | (702[M+H]⁺) |
| 88 | S88 | 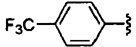 | 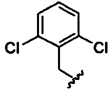 | 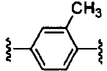 | Se | H | CH₃ | CH₂CH₃ | (750[M+H]⁺) |
| 89 | S89 | 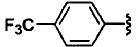 | 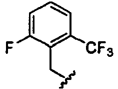 | 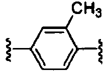 | S | H | H | CH₂CH₃ | (706[M+H]⁺) |
| 90 | S90 | 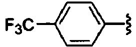 | 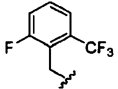 | 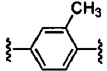 | Se | H | H | CH₂CH₃ | (754[M+H]⁺) |
| 91 | S91 | 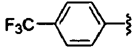 | 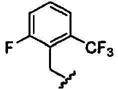 | 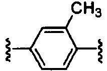 | S | CH₃ | CH₃ | CH₂CH₃ | (734[M+H]⁺) |
| 92 | S92 | 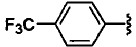 | 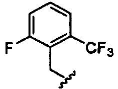 | 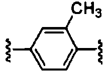 | Se | CH₃ | CH₃ | CH₂CH₃ | (782[M+H]⁺) |
| 93 | S93 | 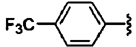 | 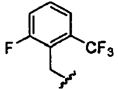 | 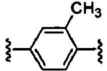 | S | H | CH₂CH₃ | CH₂CH₃ | (734[M+H]⁺) |
| 94 | S94 | 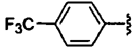 | 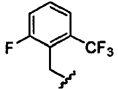 | 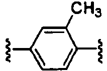 | Se | H | CH₂CH₃ | CH₂CH₃ | (782[M+H]⁺) |
| 95 | S95 | 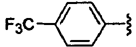 | 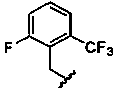 | 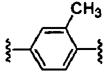 | S | H | CH₃ | CH₂CH₃ | (720[M+H]⁺) |
| 96 | S96 | 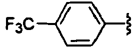 | 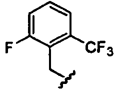 | 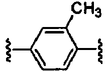 | Se | H | CH₃ | CH₂CH₃ | (768[M+H]⁺) |
| 97 | S97 | 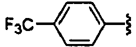 | 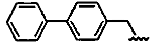 | 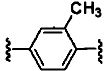 | S | H | H | CH₂CH₃ | (696[M+H]⁺) |
| 98 | S97 | 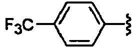 | 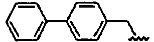 | 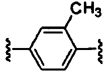 | Se | H | H | CH₂CH₃ | (744[M+H]⁺) |
| 99 | S99 | 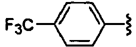 | 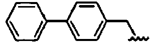 | 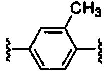 | S | CH₃ | CH₃ | CH₂CH₃ | (724[M+H]⁺) |
| 100 | S100 | 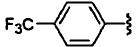 | 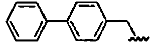 | 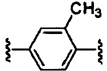 | Se | CH₃ | CH₃ | CH₂CH₃ | (772[M+H]⁺) |
| 101 | S101 | 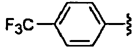 | 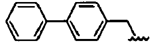 | 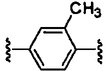 | S | H | CH₂CH₃ | CH₂CH₃ | (724[M+H]⁺) |
| 102 | S102 | 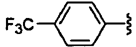 | 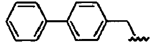 | 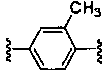 | Se | H | CH₂CH₃ | CH₂CH₃ | (772[M+H]⁺) |
| 103 | S103 | 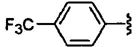 | 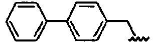 | 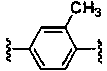 | S | H | CH₃ | CH₂CH₃ | (710[M+H]⁺) |
| 104 | S104 | 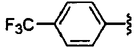 | 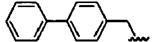 | 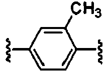 | Se | H | CH₃ | CH₂CH₃ | (758[M+H]⁺) |
| 105 | S105 | 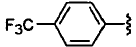 | 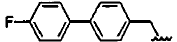 | 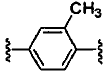 | S | H | H | CH₂CH₃ | (713[M+H]⁺) |
| 106 | S106 | 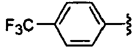 | 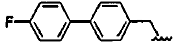 | 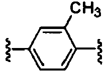 | Se | H | H | CH₂CH₃ | (762[M+H]⁺) |
| 107 | S107 | 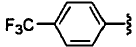 | 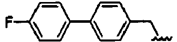 | 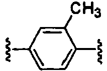 | S | CH₃ | CH₃ | CH₂CH₃ | (742[M+H]⁺) |
| 108 | S108 | 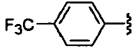 | 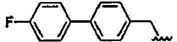 | 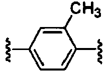 | Se | CH₃ | CH₃ | CH₂CH₃ | (790[M+H]⁺) |
| 109 | S109 | 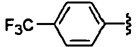 | 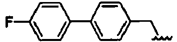 | 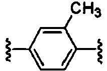 | S | H | CH₂CH₃ | CH₂CH₃ | (742[M+H]⁺) |
| 110 | S110 | 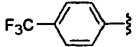 | 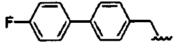 | 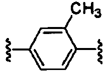 | Se | H | CH₂CH₃ | CH₂CH₃ | (790[M+H]⁺) |
| 111 | S111 | 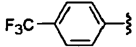 | 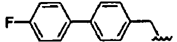 | 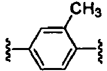 | S | H | CH₃ | CH₂CH₃ | (728[M+H]⁺) |
| 112 | S112 | 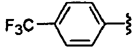 | 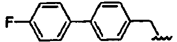 | 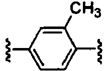 | Se | H | CH₃ | CH₂CH₃ | (776[M+H]⁺) |
| 113 | S113 | 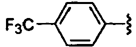 | 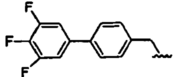 | 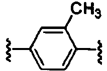 | S | H | H | CH₂CH₃ | (750[M+H]⁺) |
| 114 | S114 | 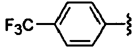 | 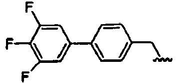 | 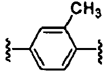 | Se | H | H | CH₂CH₃ | (798[M+H]⁺) |
| 115 | S115 | 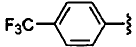 | 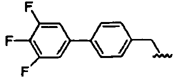 | 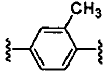 | S | CH₃ | CH₃ | CH₂CH₃ | (778[M+H]⁺) |
| 116 | S116 | 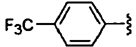 | 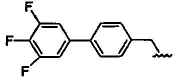 | 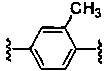 | Se | CH₃ | CH₃ | CH₂CH₃ | (826[M+H]⁺) |
| 117 | S117 | 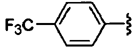 | 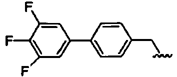 | 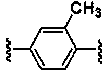 | S | H | CH₂CH₃ | CH₂CH₃ | (778[M+H]⁺) |
| 118 | S118 | 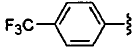 | 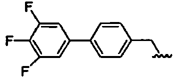 | 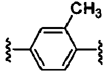 | Se | H | CH₂CH₃ | CH₂CH₃ | (826[M+H]⁺) |
| 119 | S119 | 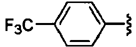 | 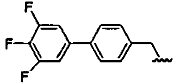 | 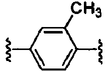 | S | H | CH₃ | CH₂CH₃ | (764[M+H]⁺) |
| 120 | S120 | 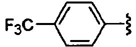 | 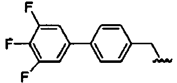 | 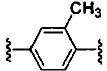 | Se | H | CH₃ | CH₂CH₃ | (812[M+H]⁺) |
| 121 | S121 | 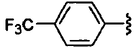 | 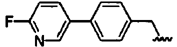 | 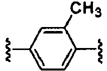 | S | H | H | CH₂CH₃ | (715[M+H]⁺) |
| 122 | S122 | 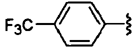 | 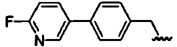 | 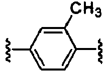 | Se | H | H | CH₂CH₃ | (763[M+H]⁺) |
| 123 | S123 | 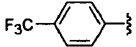 | 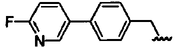 | 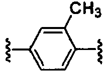 | S | CH₃ | CH₃ | CH₂CH₃ | (743[M+H]⁺) |
| 124 | S124 | 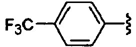 | 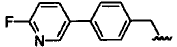 | 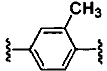 | Se | CH₃ | CH₃ | CH₂CH₃ | (791 [M+H]⁺) |
| 125 | S125 | 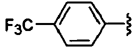 | 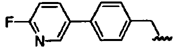 | 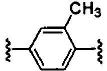 | S | H | CH₂CH₃ | CH₂CH₃ | (743[M+H]⁺) |
| 126 | S126 | 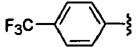 | 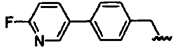 | 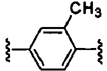 | Se | H | CH₂CH₃ | CH₂CH₃ | (791 [M+H]⁺) |
| 127 | S127 | 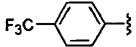 | 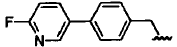 | 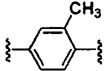 | S | H | CH₃ | CH₂CH₃ | (729[M+H]⁺) |
| 128 | S128 | 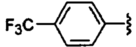 | 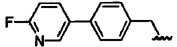 | 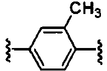 | Se | H | CH₃ | CH₂CH₃ | (777[M+H]⁺) |
| 129 | S129 | 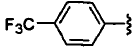 | 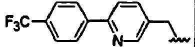 | 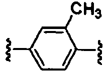 | S | H | H | CH₂CH₃ | (765[M+H]⁺) |
| 130 | S130 | 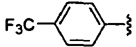 | 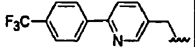 | 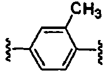 | Se | H | H | CH₂CH₃ | (813[M+H]⁺) |
| 131 | S131 | 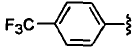 | 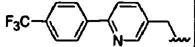 | 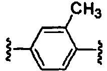 | S | CH₃ | CH₃ | CH₂CH₃ | (778[M+H]⁺) |
| 132 | S132 | 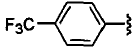 | 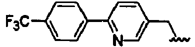 | 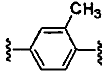 | Se | CH₃ | CH₃ | CH₂CH₃ | (826[M+H]⁺) |
| 133 | S133 | 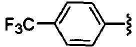 | 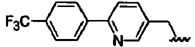 | 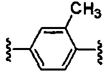 | S | H | CH₂CH₃ | CH₂CH₃ | (778[M+H]⁺) |
| 134 | S134 | 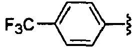 | 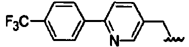 | 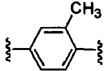 | Se | H | CH₂CH₃ | CH₂CH₃ | (826[M+H]⁺) |
| 135 | S135 | 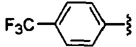 | 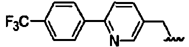 | 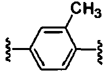 | S | H | CH₃ | CH₂CH₃ | (764[M+H]⁺) |
| 136 | S136 | 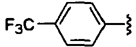 | 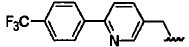 | 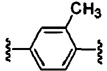 | Se | H | CH₃ | CH₂CH₃ | (812[M+H]⁺) |

### [Example 137] Preparation of Compound S137

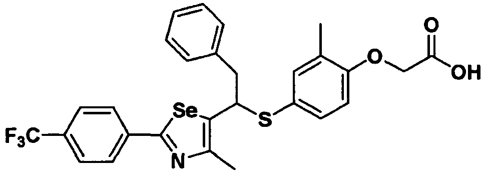

### [Step F]

Compound S49 (618 mg, 1 mmol) was mixed well with THF (15 mL) and water (10 mL) and 2.0 M lithium hydroxide aqueous solution (0.6 mL) was slowly added at 0 °C. After further stirring for 60 minutes at 0 °C, 0.5 M NaHSO₄ (2.5 mL) was added after the reaction was completed. Then, the organic solvent was extracted with brine and ethyl acetate and distilled under reduced pressure after filtration. Purification of the residue by LH-20 column chromatography yielded the target compound (566 mg, yield: 96 %). (FABMS: 592 [M+H]⁺).

### [Example 138] Preparation of Compound S138

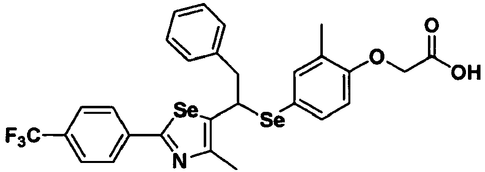

### [Step F]

Compound S50 (665 mg, 1 mmol) was mixed well with THF (15 mL) and water (10 mL) and 2.0 M lithium hydroxide aqueous solution (0.6 mL) was slowly added at 0 °C. After further stirring for 60 minutes at 0 °C, 0.5 M NaHSO₄ (2.5 mL) was added after the reaction was completed. Then, the organic solvent was extracted with brine and ethyl acetate and distilled under reduced pressure after filtration. Purification of the residue by LH-20 column chromatography yielded the target compound (605 mg, yield: 95 %). (FABMS: 640 [M+H]⁺).

### [Examples 139 to 288]

Compounds S139 to S288 listed in Table 3 were prepared according to the procedure of Examples 87 and 88. MS analysis result is given in the table.

**[Table 3]**

| | | | | | | | | | |
|---|---|---|---|---|---|---|---|---|---|
| 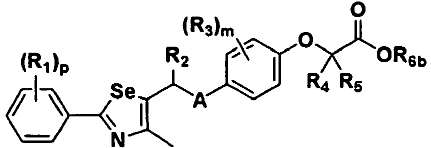 | | | | | | | | | |

| **Example** | **Compound No.** | 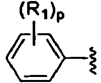 | R₂ | 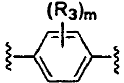 | A | R₄ | R₅ | R_{6b} | FABMS: |
|---|---|---|---|---|---|---|---|---|---|
| 139 | S139 | 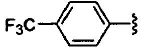 | 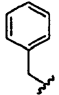 | 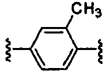 | S | CH₃ | CH₃ | H | (620[M+H]⁺) |
| 140 | S140 | 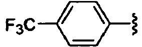 | 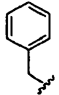 | 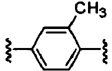 | Se | CH₃ | CH₃ | H | (668[M+H]⁺) |
| 141 | S141 | 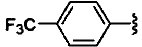 | 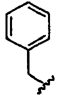 | 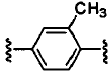 | S | H | CH₂CH₃ | H | (620[M+H]⁺) |
| 142 | S142 | 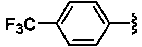 | 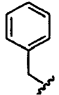 | 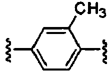 | Se | H | CH₂CH₃ | H | (668[M+H]⁺) |
| 143 | S143 | 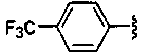 | 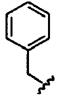 | 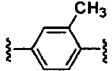 | S | H | CH₃ | H | (606[M+H]⁺) |
| 144 | S144 | 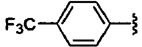 | 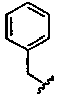 | 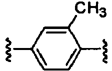 | Se | H | CH₃ | H | (654[M+H]⁺) |
| 145 | S145 | 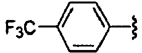 | 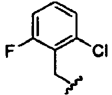 | 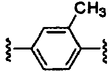 | S | H | H | H | (644[M+H]⁺) |
| 146 | S146 | 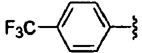 | 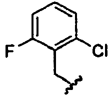 | 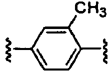 | Se | H | H | H | (792[M+H]⁺) |
| 147 | S147 | 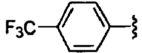 | 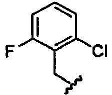 | 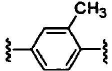 | S | CH₃ | CH₃ | H | (672[M+H]⁺) |
| 148 | S148 | 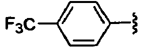 | 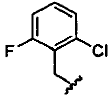 | 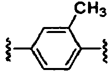 | Se | CH₃ | CH₃ | H | (718[M+H]⁺) |
| 149 | S149 | 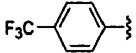 | 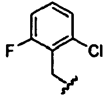 | 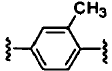 | S | H | CH₂CH₃ | H | (672[M+H]⁺) |
| 150 | S150 | 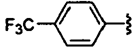 | 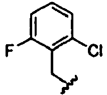 | 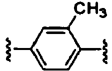 | Se | H | CH₂CH₃ | H | (718[M+H]⁺) |
| 151 | S151 | 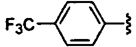 | 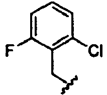 | 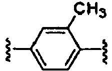 | S | H | CH₃ | H | (658[M+H]⁺) |
| 152 | S152 | 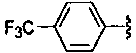 | 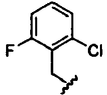 | 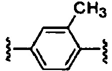 | Se | H | CH₃ | H | (706[M+H]⁺) |
| 153 | S153 | 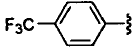 | 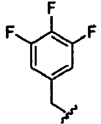 | 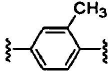 | S | H | H | H | (646[M+H]⁺) |
| 154 | S154 | 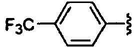 | 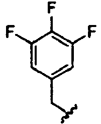 | 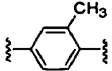 | Se | H | H | H | (694[M+H]⁺) |
| 155 | S155 | 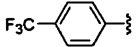 | 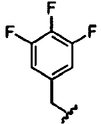 | 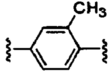 | S | CH₃ | CH₃ | H | (674[M+H]⁺) |
| 156 | S156 | 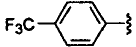 | 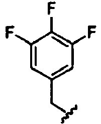 | 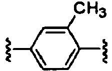 | Se | CH₃ | CH₃ | H | (722[M+H]⁺) |
| 157 | S157 | 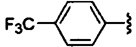 | 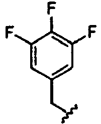 | 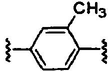 | S | H | CH₂CH₃ | H | (674[M+H]⁺) |
| 158 | S158 | 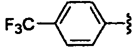 | 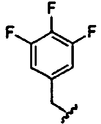 | 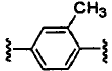 | Se | H | CH₂CH₃ | H | (722[M+H]⁺) |
| 159 | S159 | 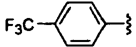 | 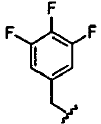 | 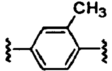 | S | H | CH₃ | H | (660[M+H]⁺) |
| 160 | S160 | 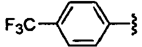 | 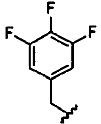 | 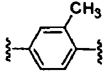 | Se | H | CH₃ | H | (708[M+H]⁺) |
| 161 | S161 | 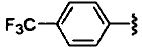 | 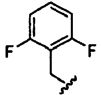 | 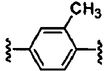 | S | H | H | H | (628[M+H]⁺) |
| 162 | S162 | 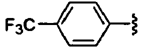 | 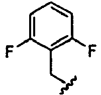 | 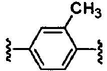 | Se | H | H | H | (676[M+H]⁺) |
| 163 | S163 | 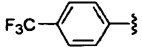 | 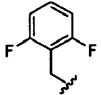 | 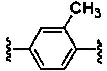 | S | CH₃ | CH₃ | H | (656[M+H]⁺) |
| 164 | S164 | 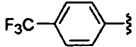 | 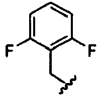 | 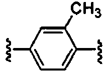 | Se | CH₃ | CH₃ | H | (704[M+H]⁺) |
| 165 | S165 | 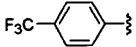 | 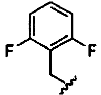 | 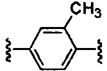 | S | H | CH₂CH₃ | H | (656[M+H]⁺) |
| 166 | S166 | 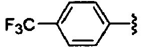 | 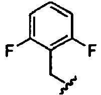 | 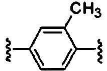 | Se | H | CH₂CH₃ | H | (704[M+H]⁺) |
| 167 | S167 | 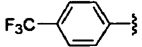 | 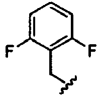 | 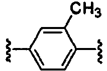 | S | H | CH₃ | H | (642[M+H]⁺) |
| 168 | S168 | 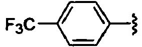 | 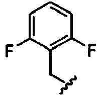 | 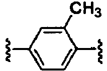 | Se | H | CH₃ | H | (690[M+H]⁺) |
| 169 | S169 | 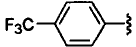 | 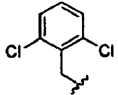 | 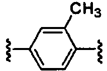 | S | H | H | H | (660[M+H]⁺) |
| 170 | S170 | 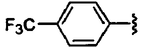 | 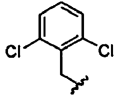 | 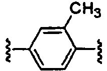 | Se | H | H | H | (706[M+H]⁺) |
| 171 | S171 | 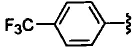 | 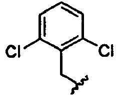 | 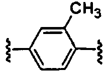 | S | CH₃ | CH₃ | H | (668[M+H]⁺) |
| 172 | S172 | 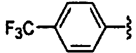 | 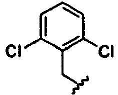 | 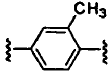 | Se | CH₃ | CH₃ | H | (736[M+H]⁺) |
| 173 | S173 | 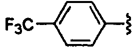 | 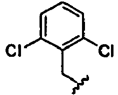 | 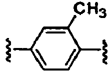 | S | H | CH₂CH₃ | H | (768[M+H]⁺) |
| 174 | S174 | 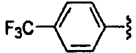 | 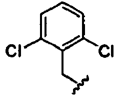 | 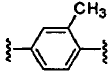 | Se | H | CH₂CH₃ | H | (736[M+H]⁺) |
| 175 | S175 | 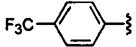 | 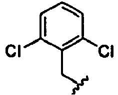 | 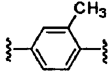 | S | H | CH₃ | H | (674[M+H]⁺) |
| 176 | S176 | 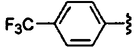 | 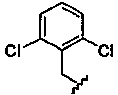 | 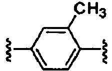 | Se | H | CH₃ | H | (722[M+H]⁺) |
| 177 | S177 | 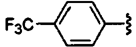 | 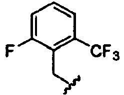 | 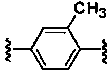 | S | H | H | H | (678[M+H]⁺) |
| 178 | S178 | 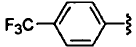 | 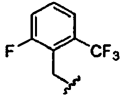 | 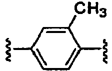 | Se | H | H | H | (726[M+H]⁺) |
| 179 | S179 | 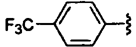 | 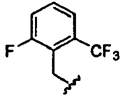 | 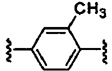 | S | CH₃ | CH₃ | H | (706[M+H]⁺) |
| 180 | S180 | 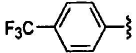 | 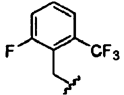 | 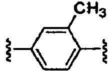 | Se | CH₃ | CH₃ | H | (754[M+H]⁺) |
| 181 | S181 | 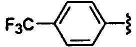 | 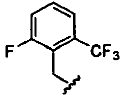 | 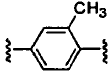 | S | H | CH₂CH₃ | H | (706[M+H]⁺) |
| 182 | S182 | 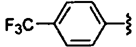 | 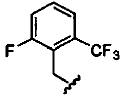 | 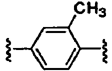 | Se | H | CH₂CH₃ | H | (754[M+H]⁺) |
| 183 | S183 | 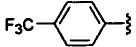 | 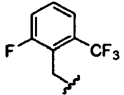 | 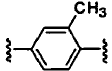 | S | H | CH₃ | H | (692[M+H]⁺) |
| 184 | S184 | 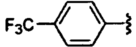 | 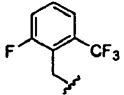 | 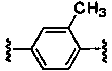 | Se | H | CH₃ | H | (740[M+H]⁺) |
| 185 | S185 | 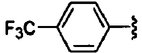 | H | 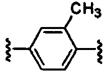 | S | H | H | H | (502[M+H]⁺) |
| 186 | S186 | 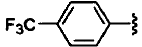 | H | 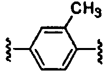 | Se | H | H | H | (550[M+H]⁺) |
| 187 | S187 | 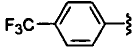 | H | 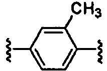 | S | CH₃ | CH₃ | H | (530[M+H]⁺) |
| 188 | S188 | 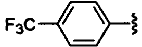 | H | 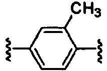 | Se | CH₃ | CH₃ | H | (578[M+H]⁺) |
| 189 | S189 | 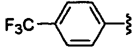 | H | 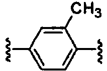 | S | H | CH₂CH₃ | H | (530[M+H]⁺) |
| 190 | S190 | 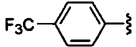 | H | 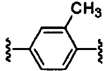 | Se | H | CH₂CH₃ | H | (578[M+H)⁺) |
| 191 | S191 | 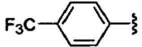 | H | 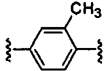 | S | H | CH₃ | H | (516[M+H]⁺) |
| 192 | S192 | 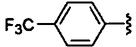 | H | 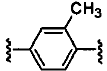 | Se | H | CH₃ | H | (564[M+H]⁺) |
| 193 | S193 | 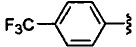 | 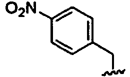 | 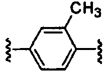 | S | H | H | H | (637[M+H]⁺) |
| 194 | S194 | 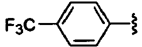 | 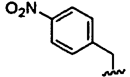 | 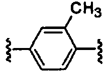 | Se | H | H | H | (684[M+H]⁺) |
| 195 | S195 | 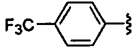 | 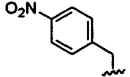 | 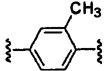 | S | CH₃ | CH₃ | H | (665[M+H]⁺) |
| 196 | S196 | 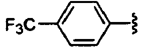 | 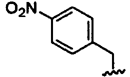 | 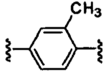 | Se | CH₃ | CH₃ | H | (713[M+H]⁺) |
| 197 | S197 | 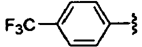 | 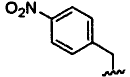 | 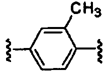 | S | H | CH₂CH₃ | H | (665[M+H]⁺) |
| 198 | S198 | 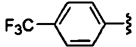 | 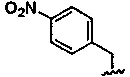 | 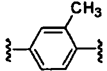 | Se | H | CH₂CH₃ | H | (713[M+H]⁺) |
| 199 | S199 | 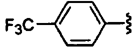 | 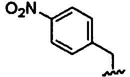 | 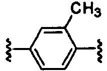 | S | H | CH₃ | H | (651[M+H]⁺) |
| 200 | S200 | 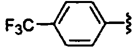 | 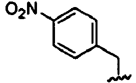 | 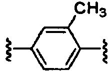 | Se | H | CH₃ | H | (697[M+H]⁺) |
| 201 | S201 | 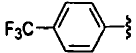 | 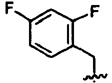 | 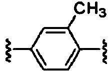 | S | H | H | H | (628[M+H]⁺) |
| 202 | S202 | 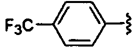 | 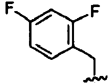 | 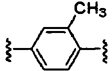 | Se | H | H | H | (675(M+H]⁺) |
| 203 | S203 | 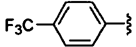 | 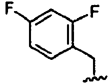 | 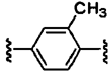 | S | CH₃ | CH₃ | H | (656[M+H]⁺) |
| 204 | S204 | 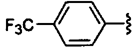 | 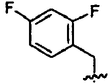 | 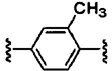 | Se | CH₃ | CH₃ | H | (704[M+H]⁺) |
| 205 | S205 | 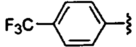 | 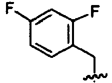 | 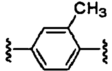 | S | H | CH₂CH₃ | H | (656[M+H]⁺) |
| 206 | S206 | 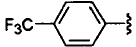 | 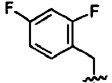 | 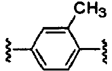 | Se | H | CH₂CH₃ | H | (704[M+H]⁺) |
| 207 | S207 | 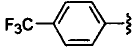 | 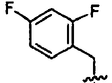 | 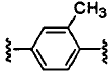 | S | H | CH₃ | H | (642[M+H]⁺) |
| 208 | S208 | 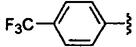 | 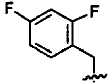 | 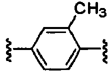 | Se | H | CH₃ | H | (690[M+H]⁺) |
| 209 | S209 | 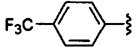 | 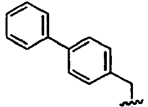 | 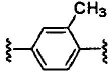 | S | H | H | H | (668[M+H]⁺) |
| 210 | S210 | 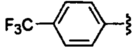 | 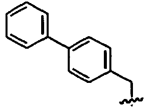 | 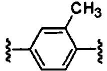 | Se | H | H | H | (716[M+H]⁺) |
| 211 | S211 | 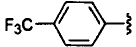 | 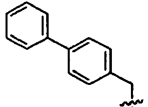 | 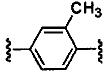 | S | CH₃ | CH₃ | H | (696[M+H]⁺) |
| 212 | S212 | 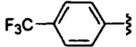 | 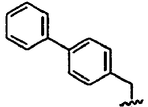 | 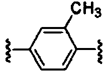 | Se | CH₃ | CH₃ | H | (744[M+H]⁺) |
| 213 | S213 | 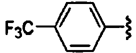 | 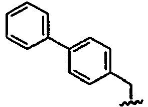 | 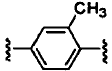 | S | H | CH₂CH₃ | H | (796[M+H]⁺) |
| 214 | S214 | 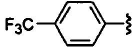 | 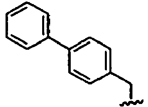 | 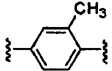 | Se | H | CH₂CH₃ | H | (744[M+H]⁺) |
| 215 | S215 | 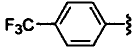 | 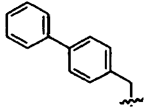 | 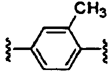 | S | H | CH₃ | H | (682[M+H]⁺) |
| 216 | S216 | 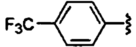 | 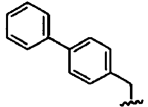 | 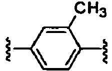 | Se | H | CH₃ | H | (730[M+H]⁺) |
| 217 | S217 | 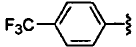 | CH₃ | 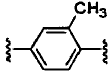 | S | H | H | H | (516[M+H]⁺) |
| 218 | S218 | 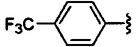 | CH₃ | 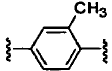 | Se | H | H | H | (563[M+H]⁺) |
| 219 | S219 | 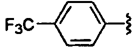 | CH₃ | 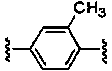 | S | CH₃ | CH₃ | H | (544[M+H]⁺) |
| 220 | S220 | 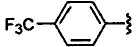 | CH₃ | 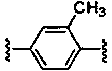 | Se | CH₃ | CH₃ | H | (592[M+H]⁺) |
| 221 | S221 | 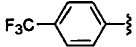 | CH₃ | 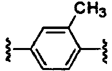 | S | H | CH₂CH₃ | H | (544[M+H]⁺) |
| 222 | S222 | 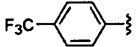 | CH₃ | 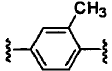 | Se | H | CH₂CH₃ | H | (592[M+H]⁺) |
| 223 | S223 | 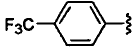 | CH₃ | 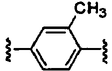 | S | H | CH₃ | H | (530[M+H]⁺) |
| 224 | S224 | 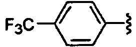 | CH₃ | 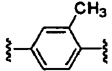 | Se | H | CH₃ | H | (577[M+H]⁺) |
| 225 | S225 | 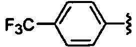 | 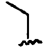 | 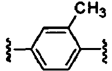 | S | H | H | H | (530[M+H]⁺) |
| 226 | S226 | 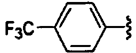 | 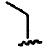 | 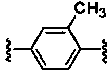 | Se | H | H | H | (577[M+H]⁺) |
| 227 | S227 | 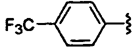 | 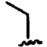 | 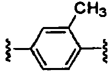 | S | CH₃ | CH₃ | H | (558[M+H]⁺) |
| 228 | S228 | 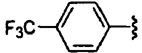 | 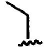 | 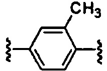 | Se | CH₃ | CH₃ | H | (606[M+H]⁺) |
| 229 | S229 | 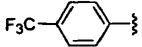 | 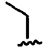 | 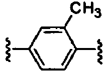 | S | H | CH₂CH₃ | H | (558[M+H]⁺) |
| 230 | S230 | 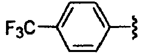 | 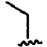 | 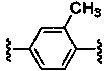 | Se | H | CH₂CH₃ | H | (606[M+H]⁺) |
| 231 | S231 | 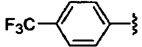 | 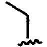 | 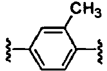 | S | H | CH₃ | H | (544[M+H]⁺) |
| 232 | S232 | 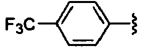 | 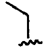 | 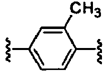 | Se | H | CH₃ | H | (592[M+H]⁺) |
| 233 | S233 | 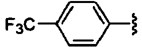 | 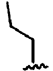 | 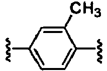 | S | H | H | H | (544[M+H]⁺) |
| 234 | S234 | 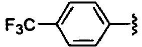 | 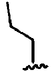 | 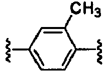 | Se | H | H | H | (592[M+H]⁺) |
| 235 | S235 | 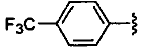 | 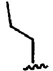 | 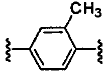 | S | H₃ | CH₃ | H | (572[M+H]⁺) |
| 236 | S236 | 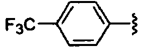 | 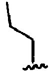 | 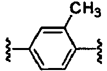 | Se | CH₃ | CH₃ | H | (620[M+H]⁺) |
| 237 | S237 | 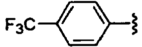 | 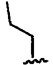 | 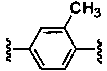 | S | H | CH₂CH₃ | H | (572[M+H]⁺) |
| 238 | S238 | 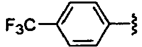 | 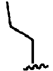 | 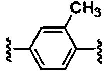 | Se | H | CH₂CH₃ | H | (620[M+H]⁺) |
| 239 | S239 | 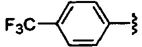 | 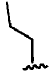 | 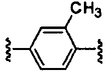 | S | H | CH₃ | H | (578[M+H]⁺) |
| 240 | S240 | 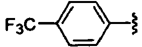 | 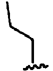 | 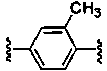 | Se | H | CH₃ | H | (606[M+H]⁺) |
| 241 | S241 | 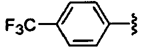 | 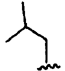 | 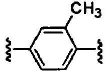 | S | H | H | H | (558[M+H]⁺) |
| 242 | S242 | 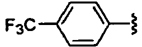 | 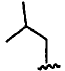 | 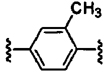 | Se | H | H | H | (606[M+H]⁺) |
| 243 | S243 | 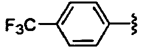 | 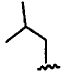 | 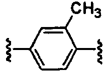 | S | CH₃ | CH₃ | H | (586[M+H]⁺) |
| 244 | S244 | 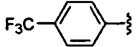 | 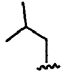 | 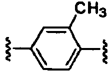 | Se | CH₃ | CH₃ | H | (634[M+H]⁺) |
| 245 | S245 | 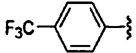 | 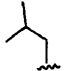 | 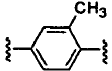 | S | H | CH₂CH₃ | H | (586[M+H]⁺) |
| 246 | S246 | 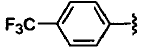 | 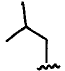 | 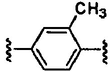 | Se | H | CH₂CH₃ | H | (634[M+H]⁺) |
| 247 | S247 | 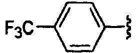 | 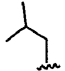 | 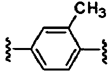 | S | H | CH₃ | H | (572[M+H]⁺) |
| 248 | S248 | 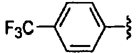 | 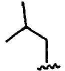 | 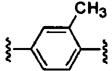 | Se | H | CH₃ | H | (620[M+H]⁺) |
| 249 | S249 | 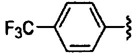 | 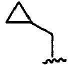 | 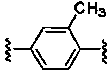 | S | H | H | H | (556[M+H]⁺) |
| 250 | S250 | 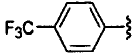 | 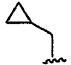 | 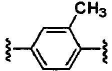 | Se | H | H | H | (604[M+H]⁺) |
| 251 | S251 | 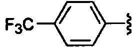 | 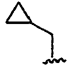 | 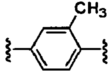 | S | CH₃ | CH₃ | H | (584[M+H]⁺) |
| 252 | S252 | 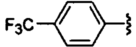 | 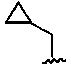 | 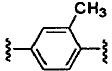 | Se | CH₃ | CH₃ | H | (632[M+H]⁺) |
| 253 | S253 | 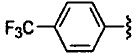 | 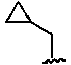 | 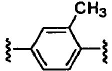 | S | H | CH₂CH₃ | H | (584[M+H]⁺) |
| 254 | S254 | 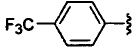 | 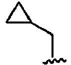 | 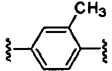 | Se | H | CH₂CH₃ | H | (632[M+H]⁺) |
| 255 | S255 | 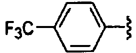 | 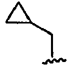 | 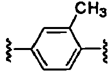 | S | H | CH₃ | H | (570[M+H]⁺) |
| 256 | S256 | 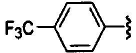 | 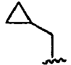 | 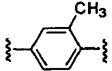 | Se | H | CH₃ | H | (618[M+H]⁺) |
| 257 | S257 | 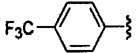 | 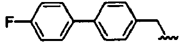 | 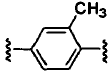 | S | H | H | H | (686[M+H]⁺) |
| 258 | S258 | 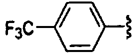 | 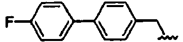 | 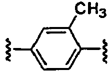 | Se | H | H | H | (734[M+H]⁺) |
| 259 | S259 | 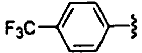 | 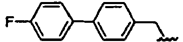 | 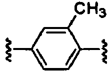 | S | CH₃ | CH₃ | H | (714[M+H]⁺) |
| 260 | S260 | 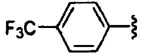 | 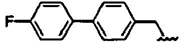 | 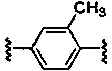 | Se | CH₃ | CH₃ | H | (762(M+H]⁺) |
| 261 | S261 | 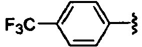 | 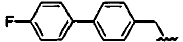 | 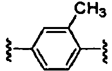 | S | H | CH₂CH₃ | H | (714[M+H]⁺) |
| 262 | S262 | 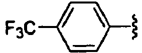 | 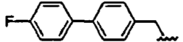 | 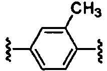 | Se | H | CH₂CH₃ | H | (762[M+H]⁺) |
| 263 | S263 | 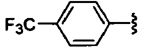 | 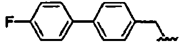 | 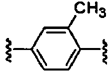 | S | H | CH₃ | H | (700[M+H]⁺) |
| 264 | S264 | 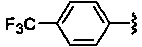 | 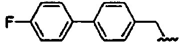 | 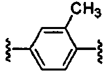 | Se | H | CH₃ | H | (748[M+H]⁺) |
| 265 | S265 | 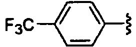 | 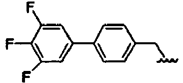 | 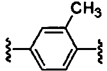 | S | H | H | H | (722[M+H]⁺) |
| 266 | S266 | 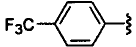 | 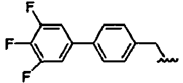 | 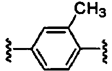 | Se | H | H | H | (770[M+H]⁺) |
| 267 | S267 | 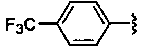 | 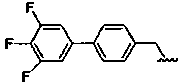 | 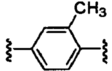 | S | CH₃ | CH₃ | H | (750[M+H]⁺) |
| 268 | S268 | 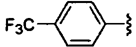 | 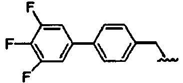 | 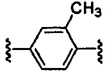 | Se | CH₃ | CH₃ | H | (798[M+H]⁺) |
| 269 | S269 | 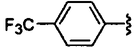 | 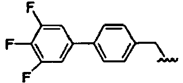 | 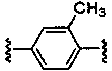 | S | H | CH₂CH₃ | H | (750[M+H]⁺) |
| 270 | S270 | 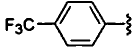 | 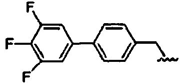 | 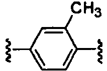 | Se | H | CH₂CH₃ | H | (798[M+H]⁺) |
| 271 | S271 | 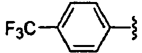 | 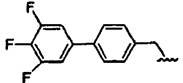 | 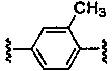 | S | H | CH₃ | H | (736[M+H]⁺) |
| 272 | S272 | 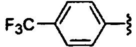 | 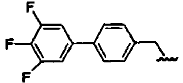 | 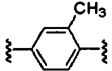 | Se | H | CH₃ | H | (784[M+H]⁺) |
| 273 | S273 | 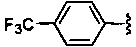 | 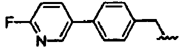 | 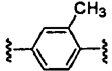 | S | H | H | H | (687[M+H]⁺) |
| 274 | S274 | 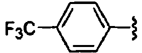 | 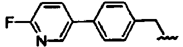 | 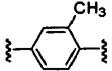 | Se | H | H | H | (735[M+H]⁺) |
| 275 | S275 | 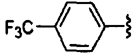 | 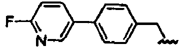 | 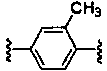 | S | CH₃ | CH₃ | H | (715[M+H]⁺) |
| 276 | S276 | 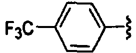 | 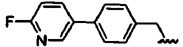 | 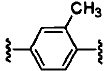 | Se | CH₃ | CH₃ | H | (763[M+H]⁺) |
| 277 | S277 | 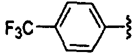 | 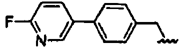 | 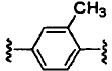 | S | H | CH₂CH₃ | H | (715[M+H]⁺) |
| 278 | S278 | 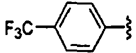 | 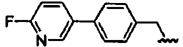 | 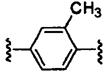 | Se | H | CH₂CH₃ | H | (763[M+H]⁺) |
| 279 | S279 | 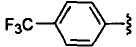 | 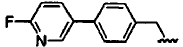 | 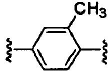 | S | H | CH₃ | H | (701[M+H]⁺) |
| 280 | S280 | 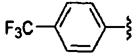 | 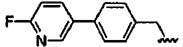 | 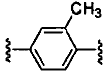 | Se | H | CH₃ | H | (749[M+H]⁺) |
| 281 | S281 | 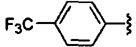 | 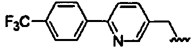 | 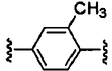 | S | H | H | H | (737[M+H]⁺) |
| 282 | S282 | 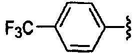 | 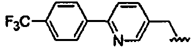 | 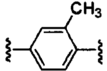 | Se | H | H | H | (785[M+H]⁺) |
| 283 | S283 | 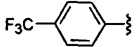 | 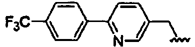 | 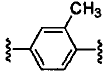 | S | CH₃ | CH₃ | H | (765[M+H]⁺) |
| 284 | S284 | 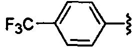 | 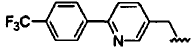 | 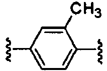 | Se | CH₃ | CH₃ | H | (813[M+H]⁺) |
| 285 | S285 | 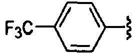 | 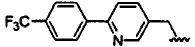 | 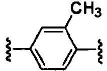 | S | H | CH₂CH₃ | H | (765[M+H]⁺) |
| 286 | S286 | 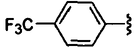 | 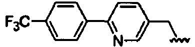 | 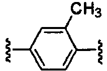 | Se | H | CH₂CH₃ | H | (813[M+H]⁺) |
| 287 | S287 | 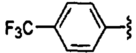 | 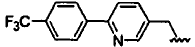 | 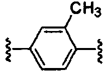 | S | H | CH₃ | H | (751[M+H]⁺) |
| 288 | S288 | 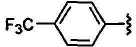 | 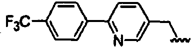 | 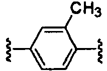 | Se | H | CH₃ | H | (799[M+H]⁺) |

### [Example 289] Preparation of Compound S289

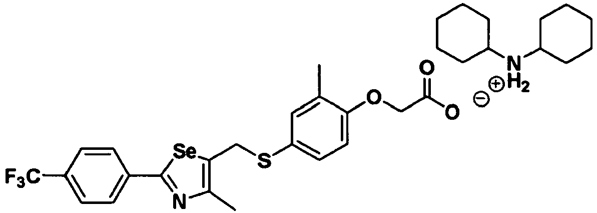

Compound S185 (500 mg, 1 mmol) was dissolved in acetonitrile (10 mL). Reaction was performed for about 20 minutes while cautiously adding dicylcohexylamine (181 mg, 1 mmol) dropwise. Then, afteradding distilled water (8 mL) and further reacting for about 10 minutes, lyophilization of the solvent yielded the target compound (674 mg, yield: 99 %). (FABMS: 683 [M+H]⁺).

### [Example 290] Preparation of Compound S290

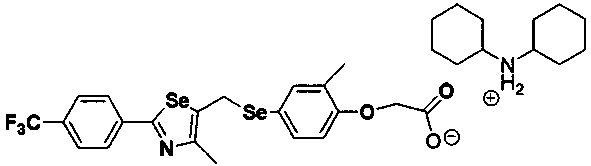

Compound S186 (590 mg, 1 mmol) was dissolved in acetonitrile (10 mL). Reaction was performed for about 20 minutes while cautiously adding dicylcohexylamine (181 mg, 1 mmol) dropwise. Then, afteradding distilled water (8 mL) and further reacting for about 10 minutes, lyophilization of the solvent yielded the target compound (768 mg, yield: 99 %). (FABMS: 731 [M+H]⁺).

### [Example 291] Preparation of Compound S291

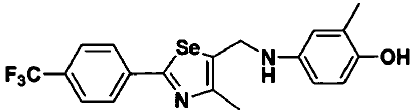

### [Step L]

CuI (10 mg, 0.05 mmol, 5 mol %), CsCO₃ (845 mg, 2.6equivalent) and 4-iodo-2-methylphenol (234 mg, 1 mmol) were added to anhydrous DMF (0.7 mL) under nitrogen atmosphere. After sealing with Teflon tape, nitrogen was filled therein. Then, after cautiously adding 2-isobutyrylcyclohexanone (34 mg, 0.2 mmol, 20 mol%) and Compound III-B-1 (320 mg, 1 equivalent) prepared in Preparation Example 5, the mixture was stirred at room temperature for 8 hours. After neutralizing the product to pH 4 using 10% HCl solution, concentration of the organic layer followed by silica gel column chromatography yielded the target compound (355 mg, yield: 79 %) (FABMS: 427 [M+H]⁺).

### [Example 292] Preparation of Compound S292

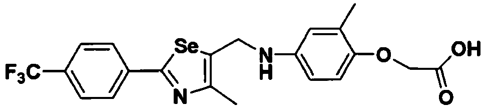

### [Steps E and F]

The target compound (348mg, yield: 82%) was prepared from Compound S291 (425 mg, 1 equivalent) according to the procedure of Examples 39 and 87 (FABMS: 485 [M+H]⁺).

### [Example 293] Preparation of Compound S293

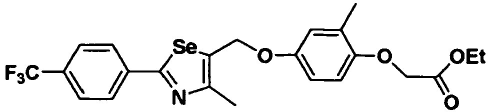

### [Step M]

Compound III-C-1 (474 mg, 1 equivalent) prepared in Preparation Example 6 was dissolved in acetonitrile (5 mL) and DMF (0.5 mL). Then, after slowly adding CsCO₃ (490 mg, 1.5 equivalents) and 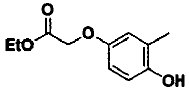 (ethyl 2-(4-hydroxy-3-methylphenoxy)acetate, 210 mg, 1 equivalent), the mixture was stirred at room temperature for 4 hours. The reaction was terminated after the disappearance of the tosyl compound was identified by TLC. Silica gel column chromatography of the organic layer yielded the target compound (435 mg, yield: 85 %) (FABMS: 514 [M+H]⁺).

### [Example 294] Preparation of Compound S294

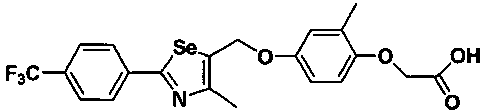

### [Step F]

The target compound (454 mg, yield: 94%) was prepared from Compound S293 (510 mg, 1 equivalent) according to the procedure of Example 87 (FABMS: 486 [M+H]⁺).

### [Example 295] Preparation of Compound S295

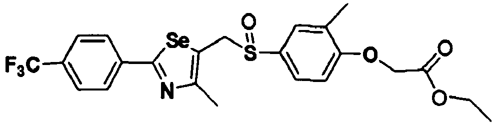

Compound S5 (530 mg, 1 mmol) was dissolved in CH₂Cl₂ (10 mL). After adding m-chloroperbenzoic acid (m-CPBA, 170 mg, 1 mmol), the temperature of the reaction mixture was maintained at 0 to 5 °C. Reaction was performed for about 1 hour at this temperature. After the reaction was completed (identified by TLC), separation of the resulting mixture by silica gel column chromatography yielded Compound S295 (485 mg, 89 %) as hazy yellow oil. (FABMS: 546[M+H]⁺).

### [Example 296] Preparation of Compound S296

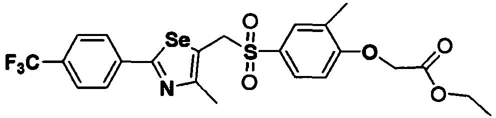

Compound S5 (530 mg, 1 mmol) was dissolved in CH₂Cl₂ (10 mL). After adding m-chloroperbenzoic acid (m-CPBA, 340 mg, 2 mmol), the temperature of the reaction mixture was maintained at 0 to 5 °C. Reaction was performed for about 2 hours at this temperature. After the reaction was completed (identified by TLC), separation of the resulting mixture by silica gel column chromatography yielded Compound S296 (516 mg, 92 %) as white solid. (FABMS: 562 [M+H]⁺).

### [Example 297] Preparation of Compound S297

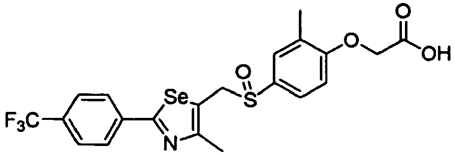

### [Step F]

The target compound (476mg, yield: 92%) was prepared from Compound S295 (545 mg, 1 equivalent) according to the procedure of Example 87 (FABMS: 518 [M+H]⁺).

### [Example 298] Preparation of Compound S298

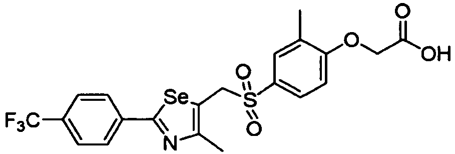

The target compound (490mg, yield: 92%) was prepared from Compound S296 (561 mg, 1 equivalent) according to the procedure of Example 87 (FABMS: 534 [M+H]⁺).

### [Test Example 1] Activity and toxicity test

PPARδ activation effect of the compound represented by Chemical Formula I according to the present invention was identified by transfection assay. Further, selectivity test for other PPAR subtypes PPARα and PPARγ, toxicity test by MTT assay, and in vivo activity test through animal experiment were carried out.

### [Transfection assay]

CV-1 cells were used for transfection assay. The cells were cultured in a 5 % CO₂ incubator at 37 °C, on a 96-well plate using DMEM medium containing 10% FBS, DBS (delipidated) and 1 % penicillin/streptomycin. Experiment was performed in four stages of cell inoculation, transfection, treatment with the compound of the present invention, and confirmation of result. The CV-1 cells inoculated onto a 96-well plate at 5,000 cells/well, and transfected 24 hours later. Transfection assay was performed using full-length PPAR plasmid DNA, reporter DNA having luciferase activity and thus capable of identifying PPAR activity, and β-galactosidase DNA which gives information about transfection efficiency. The compound of the present invention was dissolved in dimethyl sulfoxide (DMSO), diluted at different concentrations using media, and then treated to the cells. After culturing for 24 hours in an incubator, the cells were lysed using lysis buffer, and luciferase and β-galactosidase activity was measured using a luminometer and a microplate reader. The measured luciferase data were corrected using the β-galactosidase data, and were plotted to calculate the EC₅₀ value.

**[Table 4] EC₅₀ data**

| Compound No. | hPPARδ | hPPARα | hPPARγ |
|---|---|---|---|
| S185 | 0.66 nM | ia | ia |
| S186 | 4.27 nM | ia | ia |

As seen from Table 4, the compound of the present invention is highly selective for PPARδ.

The compound of the present invention exhibited an activity of 0.66 to 300 nM for PPARδ.

### [MTT assay]

Toxicity of the compound represented by Chemical Formula I according to the present invention was tested by MTT assay. MTT is a water-soluble yellow substance. But, when introduced into living cells, it is reduced to water-insoluble purple crystal by the dehydrogenase in mitochondria. Cell toxicity can be determined by dissolving MTT in dimethyl sulfoxide and measuring absorbance at 550 nm. Detailed procedure was as follows.

First, CV-1 cells were inoculated onto a 96-well plate at 5,000 cells/well. After culturing for 24 hours in a humidified 5 % CO₂ incubator at 37 °C, the compound of the present invention (Compound S185) was treated to the cultured CV-1 cells at different concentrations. After further culturing for 24 hours, MTT reagent was added. After culturing for about 15 minutes, the resulting purple crystal was dissolved in dimethyl sulfoxide and absorbance was measured using a microplate reader.

As a result, the compound represented by Chemical Formula I did not show toxicity at concentrations 100-1000 times higher than EC₅₀ for PPAR.

### [Animal test]

### [Obesity inhibiting effect]

In order to test in vivo effect of the compound according to the present invention, experiment was carried out using mouse. 8-week-ol C57BL/6 (SLC Co.) mice were used and feed containing 35 % fat was used to induce obesity. While giving the high-fat feed for 60 days, vehicle, Compound S185 or Compound S186 was orally administered (10 mg/kg/day). As a result, as compared to the vehicle group, the S185 group showed body weight increase of only 39 % and the S186 group showed body weight increase of only 42 %.

### [Atherosclerosis inhibiting effect]

In order to test atherosclerosis inhibiting effect of the compound according to the present invention, in vivo experiment was carried out using atherosclerosis animal model ApoE-/-, Ldlr-/- mice. While giving high-fat, high-cholesterol feed (20 % fat, 1.25 % cholesterol; AIN-93G diet), the compound of the present invention (Compound S185) was orally administered at 2 mg/kg/day. 28 days later, arterial plaque was stained using Sudan IV, and the atherosclerosis inhibiting effect was compared with the control group. As a result, the ApoE-/- mouse to which Compound S185 was administered showed an atherosclerosis inhibiting effect improved by 60 % as compared to the control group. Also, the Ldlr-/- mouse to which Compound S185 was administered showed an atherosclerosis inhibiting effect improved by 36 %.

### [Diabetes improving effect]

In order to test diabetes improving effect of the compound according to the present invention, glucose tolerance test (GTT) was carried out. To a mouse to which the test compound had been orally administered for 57 days, glucose (1.5 g/kg) was abdominally administered and change of blood glucose level was monitored. The group to which Compound S185 or S186 (10 mg/kg/day) was administered showed lower fasting glucose level as compared to the control group. Further, the group to which the compound according to the present invention was administered showed rapid decrease of glucose level within 20-40 minutes, and complete glucose clearance in 100 minutes. Incontrast, the group to which vehicle was administered did not maintain normal complete glucose even after 120 minutes. This result confirms that Compounds S185 and S186 are effective in improving diabetes.

### [Muscle endurance strengthening and muscle function improving effect]

Animal experiment was carried out in order to test muscle endurance strengthening and muscle function improving effect of the compound according to the present invention. Since muscle formation occurs mostly in the developmental stage, Compound S185 or S186 (10 mg/kg/day) was orally administered to a mouse during pregnancy, lactation or both. No difference in body weight or growth rate of the fetus was observed between the control group and the treated group. When the muscle was observed after removing the skin, the muscle of the treated group was redder than the control group. ATPase staining and immunostaining also revealed increased type I muscle fiber in the treated group. In order to confirm the effect of the change in the muscle fiber on improvement of muscle endurance and muscle function, test was performed using a treadmill. As a result, the treated group showed significantly longer running time as compared to the control group.

**[Table 5] Muscle endurance test result**

| Increase compared to control | Pregnancy | | Lactation | | Pregnancy + lactation | |
|---|---|---|---|---|---|---|
| | Time (times) | Distance (times) | Time (times) | Distance (times) | Time (times) | Distance (times) |
| **S185** | 2.3 | 2.7 | 2.1 | 2.6 | 3.7 | 3.9 |
| **S186** | 2.1 | 2.1 | 1.8 | 2.0 | 3.1 | 3.3 |

Further, improvement of muscle endurance and muscle function was confirmed when the compound according to the present invention was administered to an adult. 10-week-old C57BL/6 mouse was let to exercise while orally administering Compound S185 or S186 (10 mg/kg) . The mouse was let to exercise on a treadmill for 30 days, once a day for 30 minutes. Exercise condition was 5 minutes at 2 m/min, 5 minutes at 5 m/min, 5 minutes at 8 m/min, followed by 5 minutes at 20 m/min. At the end of the test, muscle endurance strengthening and muscle function improving effect was tested using a treadmill. As a result, the treated group sowed improvement both in exercise time and distance as compared to the control group.

### [Memory improving effect]

Animal (8-week-old C57BL/6 mouse) experiment was carried out in order to test the effect of the compound according to the present invention of treating dementia and Parkinson's disease through improvement of memory. Before performing experiment, an animal model of brain disease was established by injecting LPS into the brain by stereotaxy. Test groups were divided depending on the administration of the test compound and exercise. Exercise condition was 5 minutes at 2 m/min, 5 minutes at 5 m/min, 5 minutes at 8 m/min, followed by 5 minutes at 20 m/min. Morris water maze test was performed at the end of the test. The result is shown in the following table. It was confirmed that the compound according to the present invention and exercise are effective in treating dementia and Parkinson's disease through improvement of memory.

**[Table 6] water maze test result**

| Test groups | | Water maze test result |
|---|---|---|
| Vehicle | Exercise (x) | 32 sec |
| | Exercise (o) | 24 sec |
| **S185** | Exercise (x) | 20 sec |
| | Exercise (o) | 14 sec |
| **S186** | Exercise (x) | 27 sec |
| | Exercise (o) | 16 sec |

### [Fatty liver treating effect]

Animal (8-week-old C57BL/6 mouse) experiment was carried out in order to test fatty liver treating effect of the compound according to the present invention. Feed containing 35 % fat was used to induce fatty liver. While giving the high-fat feed for 78 days, Compound S185 was orally administered (10 mg/kg/day). At the end of the experiment, liver tissue was harvested and fixed in paraformaldehyde solution and then stained with hematoxylin and eosin. The result is shown in Fig. 1. As seen in the figure, the compound according to the present invention is effective in preventing fatty liver.

### [Industrial Applicability]

As described, the novel compound according to the present invention is effective as a ligand that activates PPAR and is useful for a pharmaceutical composition, functional food supplement composition, functional drink composition, food additive composition, functional cosmetic composition or an animal feed composition for preventing or treating fatty liver, atherosclerosis or hyperlipemia, preventing or treating hypercholesterolemia preventing or treating diabetes, preventing or treating obesity, strengthening muscle, preventing or treating muscular disease, improving endurance, improving memory, or preventing or treating dementia or Parkinson' s disease.

## Claims

1. A selenazole derivative represented by Chemical Formula I, a hydrate thereof, a solvate thereof, a stereoisomer thereof or a pharmaceutically acceptable salt thereof: 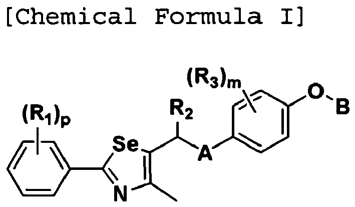 wherein
A represents O, NR, S, S(=O), S(=O)₂ or Se;
B represents hydrogen or 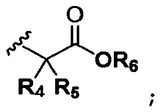 R₁ represents hydrogen, C1-C8 alkyl or halogen;
R₂ represents hydrogen, C1-C8 alkyl, 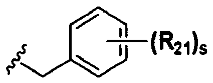 or 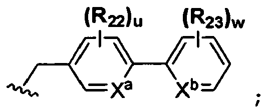 X^{a} and X^{b} independently represent CR or N;
R represents hydrogen or C1-C8 alkyl;
R₃ represents hydrogen, C1-C8 alkyl or halogen;
R₄ and R₅ independently represent hydrogen, halogen or C1-C8 alkyl; R₆ represents hydrogen, halogen, C1-C8 alkyl, C2-C7 alkenyl, allyl, an alkali metal, an alkaline earth metal or a pharmaceutically acceptable organic salt;
R₂₁, R₂₂, and R₂₃ independently represent hydrogen, halogen, CN, NO₂, C1-C7 alkyl, C6-C12 aryl, C3-C12 heteroaryl containing one or more heteroatom(s) selected from N, O and S, 5- to 7-membered heterocycloalkyl or C1-C7 alkoxy;
m represents an integer from 1 to 4;
p represents an integer from 1 to 5;
s represents an integer from 1 to 5;
u represents an integer from 1 to 3;
w represents an integer from 1 to 4; and
the alkyl and alkoxy of R₁, R₃, R₄, R₅, R₆, R₂₁, R₂₂ and R₂₃ may be further substituted with one or more halogen, C3-C7 cycloalkyl or C1-C5 alkylamine.

2. The selenazole derivative according to claim 1, a hydrate thereof, a solvate thereof, a stereoisomer thereof or a pharmaceutically acceptable salt thereof, wherein:
R₁ represents hydrogen, C1-C5 alkyl substituted with one or more fluorine, or fluorine;
R₂ represents hydrogen, C1-C8 alkyl, 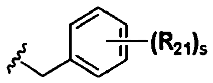 or 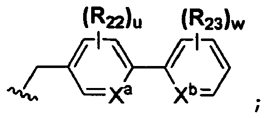
X^{a} and X^{b} independently represent CR or N;
R represents hydrogen or C1-C8 alkyl;
R₃ represents hydrogen, C1-C5 alkyl substituted or unsubstituted with halogen, or halogen;
R₄ and R₅ independently represent hydrogen, C1-C5 alkyl substituted or unsubstituted with halogen;
R₆ represents hydrogen, C1-C8 alkyl, halogen, allyl, C2-C7 alkenyl, a pharmaceutically acceptable organic salt, an alkali metal or an alkaline earth metal; and
R₂₁, R₂₂ and R₂₃ independently represent hydrogen, halogen, CN, NO₂, C1-C7 alkyl substituted or unsubstituted with halogen, C6-C12 aryl, C3-C12 heteroaryl containing one or more heteroatom (s) selected from N, O and S, 5- to 7-membered heterocycloalkyl, or C1-C5 alkoxy substituted or unsubstituted with halogen.

3. The selenazole derivative according to claim 1 which is represented by Chemical Formula IV, a hydrate thereof, a solvate thereof, a stereoisomer thereof or a pharmaceutically acceptable salt thereof: 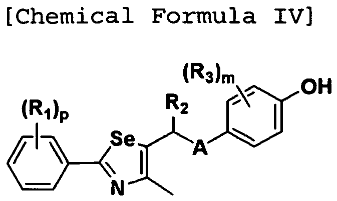 wherein
A represents O, NR, S or Se; and
R₁, R₂, R₃, m and p are the same as defined in Chemical Formula I in claim 1.

4. The selenazole derivative according to claim 1 which is represented by Chemical Formula VII, a hydrate thereof, a solvate thereof, a stereoisomer thereof or a pharmaceutically acceptable salt thereof: 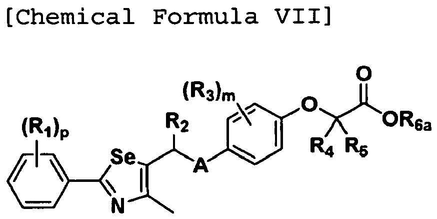 wherein
A, R₁, R₂, R₃, R₄, R₅, m and p are the same as defined in Chemical Formula I in claim 1; and
R₆ₐ represents C1-C8 alkyl or allyl.

5. The selenazole derivative according to claim 1 which is represented by Chemical Formula VIII, a hydrate thereof, a solvate thereof, a stereoisomer thereof or a pharmaceutically acceptable salt thereof: 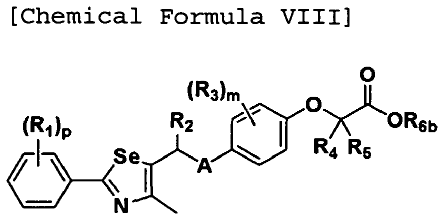 wherein
A, R₁, R₂, R₃, R₄, R₅, m and p are the same as defined in Chemical Formula I in claim 1; and
R_{6b} represents hydrogen, an alkali metal, an alkaline earth metal or a pharmaceutically acceptable organic salt.

6. A method for preparing the selenazole derivative represented by Chemical Formula I according to claim 1, comprising:
reacting a compound represented by Chemical Formula II with a Grignard reagent and then with an organolithium compound;
subsequently adding sulfur (S) or selenium (Se) powder; and
subsequently reacting with a compound represented by Chemical Formula III to prepare a compound represented by Chemical Formula IV: 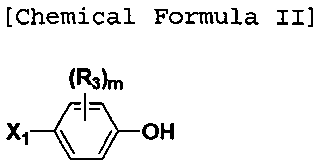 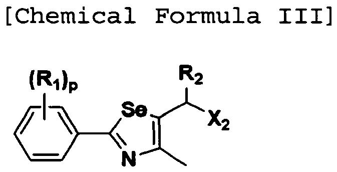 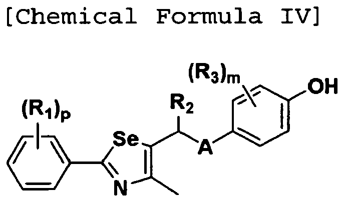
wherein A represents O, NR, S or Se; R₁, R₂, R₃, m and p are the same as defined in Chemical Formula I in claim 1; X₁ represents bromine or iodine; and X₂ represents chlorine, bromine, iodine or other leaving group suitable for nucleophilic substitution.

7. A method for preparing the selenazole derivative represented by Chemical Formula I according to claim 1, comprising:
reacting a compound represented by Chemical Formula II with a Grignard reagent and then with an organolithium compound;
subsequently adding sulfur (S) or selenium (Se) powder;
subsequently reacting with a compound represented by Chemical Formula III-A to prepare a compound represented by Chemical Formula IV-A; and
protecting the phenol group of the compound represented by Chemical Formula IV-A with an alkylsilyl group, treating the α-proton of the resulting thio- or selenoether compound with a strong base, adding a compound represented by Chemical Formula VI and then deprotecting to prepare a compound represented by Chemical Formula IV-B: 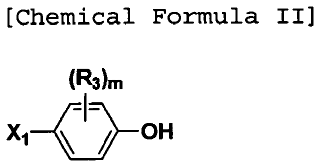 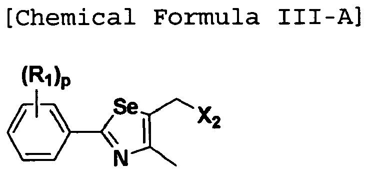 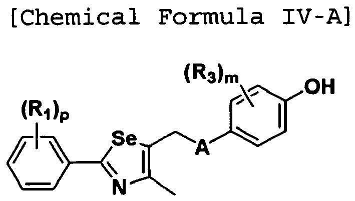
[Chemical Formula VI] **X₃-R₂** 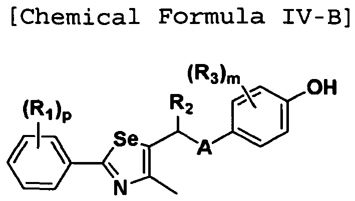 wherein A represents O, NR, S or Se; R₂ represents 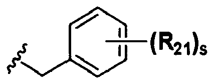 or 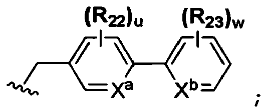 R₁, R₃, R₂₁, R₂₂, R₂₃, X^{a}, X^{b}, R, m, p, s, u and w are the same as defined in Chemical Formula I in claim 1; X₁ represents bromine or iodine; and X₂ and X₃ independently represent chlorine, bromine, iodine or other leaving group.

8. A method for preparing the selenazole derivative represented by Chemical Formula I according to claim 1, comprising:
reacting a compound represented by Chemical Formula IV-A with a Grignard reagent;
subsequently treating the α-proton of the resulting thio- or selenoether compound with a strong base; and
reacting with a compound represented by Chemical Formula VI to prepare a compound represented by Chemical Formula IV-B: 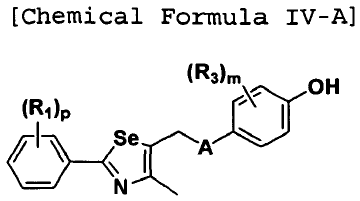
[Chemical Formula VI] X₃-R₂ 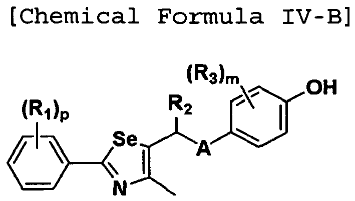 wherein A represents O, NR, S or Se; R₂ represents 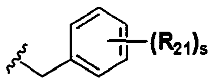 or 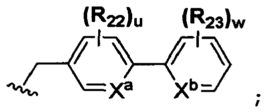 R₁, R₃, R₂₁, R₂₂, R₂₃, X^{a}, X^{b}, R, m, p, s, u and w are the same as defined in Chemical Formula I in claim 1; and X₃ represents chlorine, bromine, iodine or other leaving group.

9. A method for preparing the selenazole derivative represented by Chemical Formula I according to claim 1, comprising:
reacting a compound represented by Chemical Formula II with a compound represented by Chemical Formula III-B in the presence of copper iodide (CuI) and 2-isobutyrylcyclohexanone to prepare a compound represented by Chemical Formula IV-C: 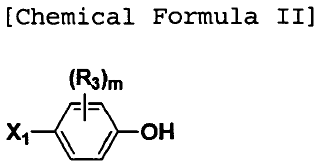 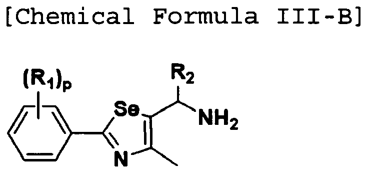 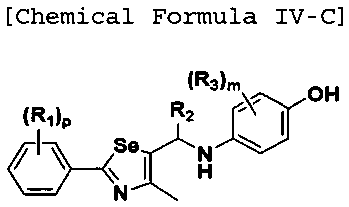
wherein R₁, R₂, R₃, m and p are the same as defined in Chemical Formula I in claim 1; and X₁ represents bromine or iodine.

10. A method for preparing the selenazole derivative represented by Chemical Formula I according to claim 1, comprising:
reacting a compound represented by Chemical Formula IV with alkyl halogen acetate or alkyl halogen acetic acid alkyl ester to prepare an ester compound represented by Chemical Formula VII: 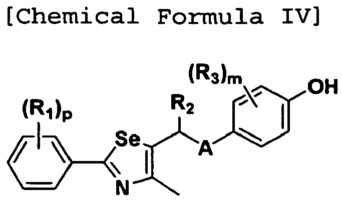 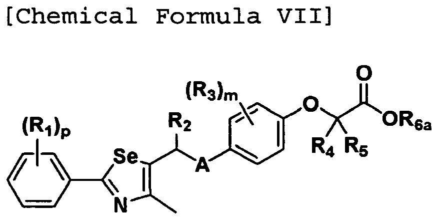
wherein A, R₁, R₂, R₃, R₄, R₅, m and p are the same as defined in Chemical Formula I in claim 1; and R₆ₐ represents C1-C8 alkyl or allyl.

11. A method for preparing the selenazole derivative represented by Chemical Formula I according to claim 1, comprising:
reacting a compound represented by Chemical Formula X with a compound represented by Chemical Formula III-C to prepare a compound represented by Chemical Formula VII-D: 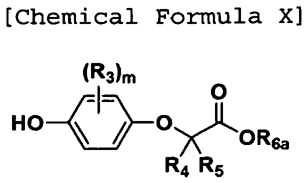 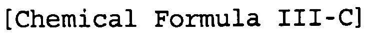 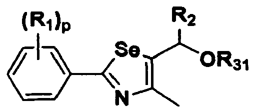 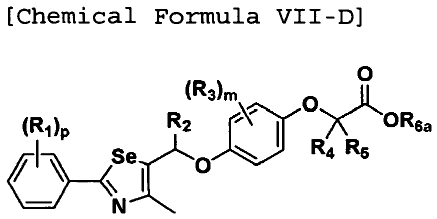
wherein R₁, R₂, R₃, R₄, R₅, m and p are the same as defined in Chemical Formula I in claim 1; R₆ₐ represents C1-C8 alkyl or allyl;
R₃₁ represents C1-C4 alkylsulfonyl or C6-C12 arylsulfonyl substituted or unsubstituted with C1-C4 alkyl.

12. The method for preparing the selenazole derivative represented by Chemical Formula I according to claim 1, according to claims 10 or 11, comprising:
hydrolyzing the ester compound represented by Chemical Formula VII to prepare a Chemical Formula VIII: 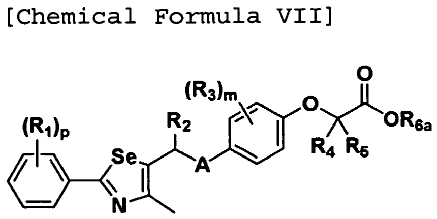 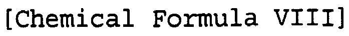 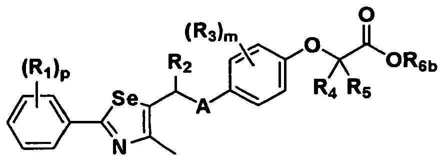
wherein A, R₁, R₂, R₃, R₄, R₅, m and p are the same as defined in Chemical Formula I in claim 1; R₆ₐ represents C1-C8 alkyl or allyl;
R_{6b} represents hydrogen, an alkali metal, an alkaline earth metal or
a pharmaceutically acceptable organic salt.

13. The method for preparing the selenazole derivative represented by Chemical Formula I according to claim 1, according to claims 10 or 11, comprising:
performing allyl ester salt substitution of the compound represented by Chemical Formula VII in an organic solvent using a tetrakis(triphenylphosphine)palladium catalyst and a metal salt to prepare a compound represented by Chemical Formula VIII: 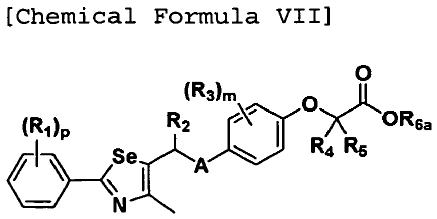 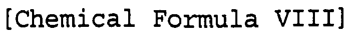 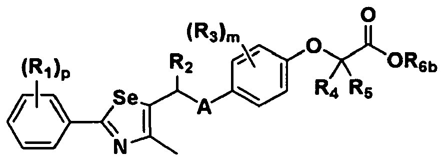
wherein A, R₁, R₂, R₃, R₄, R₅, m and p are the same as defined in Chemical Formula I in claim 1; R₆ₐ represents allyl; and R_{6b} represents an alkali metal or an alkaline earth metal.

14. A pharmaceutical composition for preventing or treating atherosclerosis or hyperlipemia, preventing or treating hypercholesterolemia, preventing or treating fatty liver, preventing or treating diabetes, preventing or treating obesity, strengthening muscle, preventing or treating muscular disease, improving endurance, improving memory, or preventing or treating dementia or Parkinson's disease comprising the selenazole derivative represented by Chemical Formula I according to claim 1, a hydrate thereof, a solvate thereof, a stereoisomer thereof or a pharmaceutically acceptable salt thereof as an effective ingredient.

15. A functional food supplement, functional drink, food additive or animal feed composition comprising the selenazole derivative represented by Chemical Formula I according to claim 1, a hydrate thereof, a solvate thereof, a stereoisomer thereof or a pharmaceutically acceptable salt thereof as an effective ingredient.

16. A functional cosmetic composition for preventing or improving obesity, preventing or improving fatty liver, strengthening muscle, preventing or improving muscular disease, or improving endurance comprising the selenazole derivative represented by Chemical Formula I according to claim 1, a hydrate thereof, a solvate thereof, a stereoisomer thereof or a pharmaceutically acceptable salt thereof as an effective ingredient.

17. A peroxisome proliferator-activated receptor (PPAR) activator composition comprising the selenazole derivative represented by Chemical Formula I according to claim 1, a hydrate thereof, a solvate thereof, a stereoisomer thereof or a pharmaceutically acceptable salt thereof as an effective ingredient.
